(19)

**Europäisches Patentamt**
**European Patent Office**
**Office européen des brevets**

(11)  **EP 4 529 916 A1**

(12)  **EUROPEAN PATENT APPLICATION**
published in accordance with Art. 153(4) EPC

(43) Date of publication:
**02.04.2025 Bulletin 2025/14**

(21) Application number: **23738053.0**

(22) Date of filing: **25.05.2023**

(51) International Patent Classification (IPC):
**A61K 9/00** (2006.01)   **A61K 9/16** (2006.01)
**A61K 47/02** (2006.01)   **A61K 47/40** (2006.01)
**A61K 47/42** (2017.01)

(52) Cooperative Patent Classification (CPC):
**A61K 9/0092; A61K 9/167; A61K 47/02;**
**A61K 47/40; A61K 47/42**

(86) International application number:
**PCT/ES2023/070337**

(87) International publication number:
**WO 2023/227817 (30.11.2023 Gazette 2023/48)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **26.05.2022 ES 202230450**

(71) Applicants:
• **Universitat Politècnica de València**
  **46022 Valencia (ES)**
• **Fundació per al Foment de la Investigació**
  **Sanitària i Biomèdica de la Communitat**
  **Valenciana**
  **(FISABIO)**
  **46010 València (ES)**
• **Consorcio Centro de Investigación Biomédica en**
  **Red**
  **28029 Madrid (ES)**

(72) Inventors:
• **DÍEZ SÁNCHEZ, Paula**
  **46022 VALENCIA (ES)**
• **ESCUDERO NOGUERA, Andrea**
  **46022 VALENCIA (ES)**
• **MARTÍNEZ MAÑEZ, Ramón**
  **46022 VALENCIA (ES)**
• **MIRA OBRADOR, Alejandro**
  **46010 VALÈNCIA (ES)**
• **IEMYTÉ, Miglé**
  **46010 VALÈNCIA (ES)**
• **AZNAR GIMENO, Elena**
  **28029 MADRID (ES)**
• **FERRER GARCÍA, María Desamparados**
  **46010 VALÈNCIA (ES)**
• **MURGUÍA IBÁÑEZ, José Ramón**
  **46022 VALENCIA (ES)**

(74) Representative: **Isern Patentes y Marcas S.L.**
  **Avda. Diagonal, 463 Bis, 2°**
  **08036 Barcelona (ES)**

(54)  **NANOPARTICLES FOR USE IN THE TREATMENT OF INFECTIONS CAUSED BY BIOFILMS**

(57)  The present invention is based on a nanodevice comprising a nanoparticle comprising a therapeutic agent, a molecular drill comprising a compound with catalytic activity, and a self-propulsion system. The molecular drill in combination with the self-propulsion system allows breaking the matrix of biofilms formed by infectious microorganisms with high efficiency and the molecular drill, in turn, allows the therapeutic agent to be released from the nanoparticle under specific conditions, preferably in the acidic environment of an infection. The invention relates to the nanodevice, to the nanodevice used in the treatment of an infection caused by biofilms, and to the use of the nanodevice to disinfect samples *in vitro* or inert materials ex *vivo.*

**EP 4 529 916 A1**

Fig. 1

**Description**

TECHNICAL FIELD OF THE INVENTION

The present invention is based on a nanodevice comprising a nanoparticle comprising a therapeutic agent, a molecular drill comprising a compound with catalytic activity, and a self-propulsion system. The molecular drill in combination with the self-propulsion system allows breaking the matrix of biofilms formed by infectious microorganisms with high efficiency and the molecular drill, in turn, allows the therapeutic agent to be released from the nanoparticle under specific conditions, preferably in the acidic environment of an infection. The invention relates to the nanodevice, to the nanodevice used in the treatment of an infection caused by biofilms, and to the use of the nanodevice to disinfect samples *in vitro* or inert materials *ex vivo.*

PRIOR ART

According to data from the World Health Organization (WHO), infectious diseases are the second leading cause of mortality, preceded only by cardiovascular diseases. Nowadays, although most acute infectious diseases such as plague and cholera have practically been eradicated as a result of the development of vaccines and antibiotic treatment, the same cannot be say with chronic infectious diseases in which the infectious agent is recalcitrant and does not respond effectively to antimicrobial treatment. In most of these infections the pathogen grows adhered to the surface of the tissue such as in chronic urinary tract infections. Similarly, these pathogenic microorganisms are also capable of growing on inert surfaces such as plastic and metallic materials of catheters or prostheses, the infection of which causes a high rate of mortality and morbidity in patients, in addition to the added cost that this entails for the health system.

It is known that antibiotics do not act effectively on these agents that cause chronic infections because said microorganisms are not in their planktonic form, but rather adhered to surfaces forming biofilms. Biofilms are complex aggregation structures formed by microorganisms for adaptation to surface colonization through the secretion of extracellular polymeric substances (EPS), and they may comprise up to hundreds of different species. The ability to form biofilms is a key virulence factor since matrix EPS facilitates immune system evasion, in addition to increasing the antibiotic resistance of the microorganism up to 1000 times. To that end, although the development of drugs to treat biofilms is of critical importance, traditional antibiotics are not effective.

Therefore, there is a need in the field to develop techniques that allow treating infections in which the pathogenic agents have formed biofilms.

BRIEF DESCRIPTION OF THE INVENTION

In a first aspect, the invention relates to a nanodevice comprising a nanoparticle, a molecular drill attached to the surface of the nanoparticle, and a self-propulsion system attached to the surface of the nanoparticle.

In a second aspect, the invention relates to a pharmaceutical composition comprising the nanodevice according to the first aspect of the invention.

In a third aspect, the invention relates to the nanodevice of the first aspect or pharmaceutical composition according to the second aspect of the invention, for use thereof as a medicinal product.

In a fourth aspect, the invention relates to the nanodevice according to the first aspect or pharmaceutical composition according to the second aspect of the invention, for use in the treatment of an infection in a subject, characterized in that the infected area in the subject comprises a biofilm produced by microorganisms that have invaded said infected area.

In a fifth aspect, the invention relates to the use of the nanodevice according to the first aspect of the invention, or of the pharmaceutical composition according to the second aspect of the invention to reduce the number of living microorganisms in a sample *in vitro* or on the surface of an inert material *ex vivo,* wherein said microorganisms have formed a biofilm, or additionally on the surface of an implanted material *in vivo.*

In a sixth aspect, the invention relates to a kit comprising the nanoparticle of the invention, the molecular drill of the invention, the therapeutic agent of the invention, and the self-propulsion system of the invention.

BRIEF DESCRIPTION OF THE FIGURES

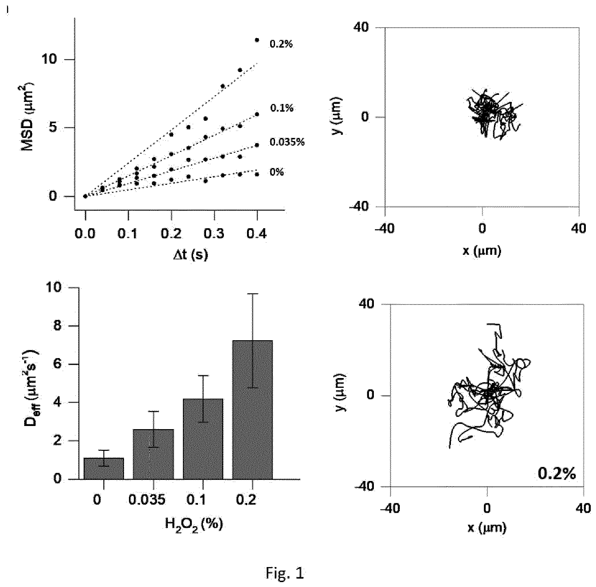

Figure 1 shows the analysis of the movement of the nanomotors, expressed as a) the mean square displacement (MSD) obtained from positions x and y of the nanomotors with respect to the time interval ($\Box t = 0.4$ s) in the presence of a range of $H_2O_2$ concentrations (0, 0.035, 0.1, and 0.2%) b) diffusion coefficient values obtained from the MSD-$\Box t$ graphs for each fuel concentration, expressed as effective coefficient (Deff, $\Box m^2\ s^{-1}$), and c) representative spatial trajectories of the nanomotors at 0 and 0.2% $H_2O_2$. (n=15, one-way ANOVA and Dunnett's multiple comparison test, *p < 0.05, ***p < 0.0001, ns not significant).

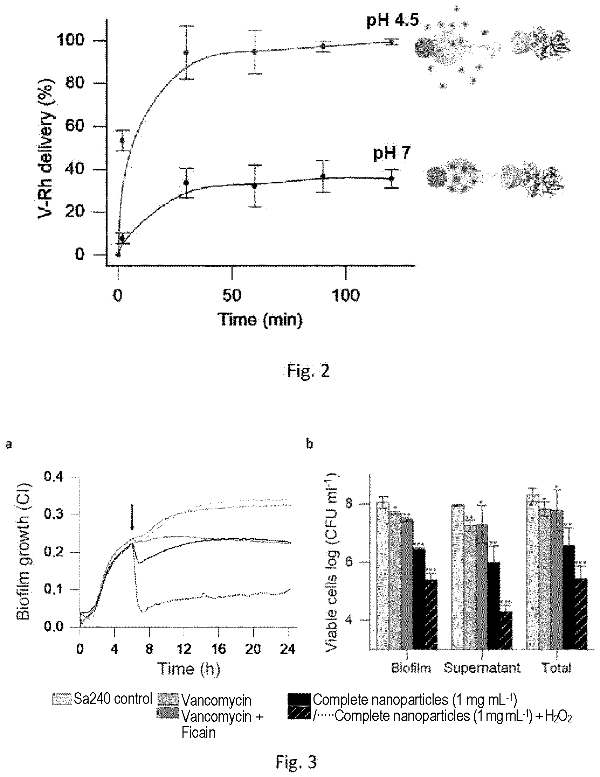

Figure 2 shows the release of the load (%) encapsulated in the nanomotor as a function of pH.

Figure 3 a) shows the monitoring of S. *aureus* Sa240 biofilm growth by means of xCELLigence RTCA SP, expressed as cell index (CI) in the presence of the components (separately and together) of the nanodevice of the invention. The black arrow indicates the moment in which the different elements were added. In ascending gray scale order, the composition containing a control Sa240 (untreated *Staphylococcus aureus* strain 240), followed by the composition comprising only the component vancomycin of the nanodevice; the composition comprising only the components vancomycin and ficin of the nanodevice which are provided separately; the composition comprising the complete nanodevice (nanoparticle with the self-propulsion system, ficin, and vancomycin) (1mg) without fuel, and dotted black line the composition comprising the complete nanodevice (nanoparticle with the self-propulsion system, ficin, and vancomycin) (1mg), in addition to 0.2% H2O2. Figure 3 b) shows cell viability expressed as logarithm of colony-forming units per milliliter, log (CFU ml-1) in the biofilm formed in the presence of each component (n=3, T-test values *p<0.05, **p<0.01, ***p<0.001, ns not significant).

DETAILED DESCRIPTION OF THE INVENTION

The authors of the invention have developed a porous nanoparticle comprising antimicrobial agents (particularly antibacterial, antifungal, or antiseptic agents) retained in the pores thereof by a molecular drill that, as a result of its size, three-dimensional structure, and arrangement, prevents the release of the antibacterial or antiseptic agents.

The molecular drill comprises a compound with catalytic activity attached to a molecular complex that allows breaking specific organic structures, particularly the composition of the matrix of a bacterial biofilm. Said molecular complex of the drill is sensitive to pH variations such that, when it comes into contact with an acidic medium, the molecular drill loses its three-dimensional structure, changes its arrangement with respect to the surface of the nanoparticle, and allows the release of the agents retained in the pores of the nanoparticle.

Furthermore, the nanoparticle comprises a self-propulsion system comprising a metal or an enzyme which, upon reacting with a specific composition, referred to as fuel in the present invention, generates physical and/or chemical changes in the medium (such as O$_2$ bubbles in the case of a self-propulsion system comprising Pt upon reacting with $H_2O_2$), providing the nanoparticle with targeted self-propulsion.

In the case of infections caused by pathogens or infectious agents that form biofilms, the medium inside the biofilm is generally acidic, and the compound with catalytic activity of the molecular drill allows breaking the structure or matrix of said biofilms. Therefore, the nanodevices of the invention are particularly useful for the controlled release of antimicrobial agents into a biofilm generated by infectious agents. Furthermore, by combining the mechanical action of the self-propulsion system with the catalytic action of the molecular drill, a synergistic effect is obtained in the ability of the nanoparticle to penetrate inside biofilms formed by infectious agents, and therefore to release a specific amount of therapeutic agent at the site of infection, and thereby treat infections formed by biofilms.

Accordingly, the invention has at least the following advantages:

- Synergistic effect between the effect of the self-propulsion system and of the catalytic activity of the molecular drill that translates into a greater amount of therapeutic agent being released in the center of an infection, and therefore the higher therapeutic efficacy of a given amount of therapeutic agent administered.

- Drastic reduction of the extracellular matrix of the biofilm formed by the infectious agent, greater than that observed with other known systems.

- Drastic reduction of cell viability (for example, about 90%) at low concentrations of therapeutic agent (for example, 13.2 mg/ml of vancomycin), which is not achieved with other known antimicrobial systems.

- Versatility of the nanodevice to be functionalized with different biomolecules in a simple manner, and therefore to treat different types of infections caused by biofilms generated by a wide range of infectious agents, as well as to disinfect inert surfaces, surgical materials, or medical materials in general. The Nanodevice of the Invention

In a first aspect, the invention relates to a nanodevice comprising a nanoparticle, a molecular drill attached to the surface of the nanoparticle, and a self-propulsion system attached to the surface of the nanoparticle.

The term "nanodevice", as it is used herein, refers to a nanometer-sized device, and generally with a spherical shape. In a particular embodiment, the nanodevice of the invention has a size of less than 1000, 900, 800, 700, 600, 500, 400, 300, 200, 100, 50 nm in the largest of its 3 dimensions, preferably less than 200 nm. In another particular embodiment, the nanodevice of the invention has a size of 100-1000 nm, 100-900, 100-800, 100-700, 100-600, 100-500, 100-400, 100-300, 100-200 nm in the largest of its 3 dimensions, preferably 100-200 nm. In another particular embodiment, the size of the nanodevice is greater than 50, 100, 200, 300, 400, 500, 600, 700, 800, 900 nm in the largest of its 3 dimensions, preferably greater than 100 nm. In another particular embodiment, the nanodevice of the invention has a size of 50, 75, 100, 105, 110, 115, 120, 125, 130, 135, 140, 145, 150, 155, 160, 165, 170, 175, 180, 185, 190, 195, 200, 210, 2220, 250, 275, 300, 325, 350, 375, 400, 450, 500, 500, 550, 600, 650, 700, 750, 800, 850, 900, 950, 1000 nm at the largest of its 3 dimensions, preferably 145 nm.

The term "the largest of its 3 dimensions", as used in the context of the nanodevice or nanoparticle of the invention, refers to the maximum length which the nanodevice or nanoparticle presents from one end to the other of its surface contour, wherein each end is defined by the furthest point from any component that is part of the nanodevice (including the molecular drill, the self-propulsion system, or the surface of the nanoparticle), or from the surface of the nanoparticle, with respect to the center of the nanodevice or nanoparticle, respectively. In the case of the nanodevice, the center thereof generally coincides with the center of the nanoparticle.

In a particular embodiment, the nanodevice has a quasi-spherical shape, and the term "the largest of its 3 dimensions" refers to its diameter.

In a particular embodiment, the term "nanoparticle", as it is used herein, refers to the term commonly used by a person skilled in the art. In a particular embodiment, it refers to a particle wherein at least one and preferably the largest of its 3 dimensions is/are equal to or less than approximately 100 nm. It generally has a spherical, semi-spherical, or hexagonal shape, so the largest of its 3 dimensions is usually considered its diameter which is therefore equal to or less than approximately 100 nm. Therefore, in a preferred embodiment, the largest of the 3 dimensions of a nanoparticle consists of its diameter. Fine particles are like nanoparticles in which the largest of the 3 dimensions is between 100 and 2,500 nm. Coarse particles are like nanoparticles in which the largest of the 3 dimensions is between 2,500 and 10,000 nm.

In a particular embodiment, the size of the nanoparticle of the nanodevice of the invention (hereinafter, the nanoparticle of the invention) has a size of less than 1000, 900, 800, 700, 600, 500, 400, 300, 250, 200, 190, 180, 170, 160, 150, 140, 130, 120, 110, 100, 90, 80, 70, 60, 50, 40, 30, 20, 10 nm in the largest of its 3 dimensions, preferably less than 140 nm. In another particular embodiment of the invention, the nanoparticle of the invention has a size of about 1000, 900, 800, 700, 600, 500, 400, 300, 250, 200, 190, 180, 170, 160, 150, 140, 130, 120, 110, 100, 90, 80, 70, 60, 50, 40, 30, 20, 10 nm in the largest of its 3 dimensions, preferably around 140 nm in the largest of its 3 dimensions. In another particular embodiment, the nanoparticle of the invention has a size of 10-1000, 50-900, 50-800, 50-700, 50-600, 50-500, 50-4450, 50-400, 50-350, 50-300, 50-250, 50-200, 75-200, 100-200, 100-150 nm in the largest of its 3 dimensions, preferably 100-250 nm.

The term "molecular drill", as it is used herein, refers to a molecular complex which comprises at least one compound capable of breaking molecular bonds of interest which, in a particular embodiment, is a compound with catalytic activity, and optionally, a subsequent molecular complex. As will be understood by one skilled in the art, the compound capable of breaking molecular bonds of interest is attached to the molecular complex, and together, they both form the molecular complex referred to as the molecular drill. The term "molecular complex", as it is used herein, refers to a molecular structure comprising at least two molecular units attached by a chemical bond, generally a *Van der Waals* bond or hydrogen bridge bonds.

The term "attachment" or "attached", as it is used in the invention in relation to the components forming the nanodevice of the invention, refers to a relationship between two components that cancels the freedom of movement of one component with respect to the other in at least one direction. Said attachment can be of a chemical type or a physical type. In the case of chemical attachments, these are covalent bonds, ionic bonds, metal bonds, *Van der Waals* bonds, or hydrogen bonds. As will be understood by one skilled in the art, said types have been listed from strongest to weakest. In the case of physical bonds, it would be a physical modification of one of the components involved in the attachment that cancels the freedom of movement of one component with respect to the other in at least one direction. Said deformation may result from the application of pressure on, or the deposition of, one or both components at the same time, such that one component is trapped in the physical structure of the other (and therefore with the freedom of movement of one component with respect to the other being canceled in at least one direction).

In one embodiment of the invention, the molecular drill comprised in the nanodevice of the invention (hereinafter molecular drill of the invention) is attached to the surface of the nanoparticle of the invention by means of a chemical bond or a physical bond; such as a coordination bond; as well as electrostatic interactions due to the electrical charges that each of them comprises. In a preferred embodiment, the molecular drill of the invention is attached to the surface of the nanoparticle of the invention by means of a chemical bond, preferably by means of a covalent bond. In a particular embodiment, the chemical bond occurs between a component of a molecule in the molecular drill and another component of a molecule on the surface of the nanoparticle. In a preferred embodiment, the chemical bond is between the molecule of an imidazole derivative, preferably benzimidazole of the molecular drill, and a silane molecule on the surface of the nanoparticle, preferably a silica nanoparticle.

The term "self-propulsion system", as it is used herein, refers to a system that generates energy which allows the movement of the component that comprises it. In the context of the self-propulsion system of the nanodevice of the invention, the self-propulsion system comprises a metal, a mineral, or an enzyme which, upon coming into contact with a specific composition, referred to as "fuel" in the context of the invention, generates a physical and/or chemical change in the environment of the nanodevice which promotes the movement of the nanodevice of the invention. Preferably, the change would be a chemical change because a chemical reaction occurs between the components, thereby generating the desired propulsion of the nanodevice. Said composition or fuel can be provided in a controlled manner to the environment in which the nanodevice of the invention is located, at the time and area of interest, such that the movement of the nanodevice of the invention can be activated at a time and in a direction of interest. As will be understood by one skilled in the art, the fuel used is specific for each type of metal or enzyme comprised in the self-propulsion system.

In one embodiment, the self-propulsion system is attached to the surface of the nanoparticle of the invention by means of a chemical or physical bond. In the case of a chemical bond, it is preferably a chemical bond such as those described in the definition of "attachment" above, between an element on the surface of the nanoparticle and the metal or a component of the enzyme of the self-propulsion system. In a preferred embodiment, said chemical bond is a covalent bond. In the case of a physical bond, it would preferably be an attachment resulting from the deposition of at least one metal or mineral of the propulsion system on the surface of the nanoparticle, preferably on a portion of the nanoparticle. Methods for attaching a metal or mineral comprised in the self-propulsion system to the surface of the nanoparticle by means of the deposition thereof are described in the section "self-propulsion system of the invention".

In a preferred embodiment, the propulsion system comprises a metal, preferably platinum (Pt), that is deposited by means of the vaporization of its atoms (in gaseous state), after being bombarded with energetic ions (plasma), on a region of the surface of the nanoparticle, preferably at a specific point of the surface of the nanoparticle.

In another embodiment, the propulsion system comprises platinum (Pt), by means of the synthesis of the platinum nanoparticle (by metallic bonding of various seeds of said element), and the subsequent attachment thereof to the nanoparticle by means of a strong covalent bond.

In a particular embodiment, the region of the surface of the nanoparticle occupied by the self-propulsion system is less than 75%, 70%, 65%, 60%, 55%, 50%, 45%, 40%, 35%, 30%, 25%, 20%, 15%, 12%, 10%, 9%, 8%, 7%, 6%, 5%, 4%, 3%, 2%, 1%, preferably less than 25%.

In a particular embodiment, the nanodevice of the invention further comprises at least one therapeutic agent in contact with the surface of the nanoparticle.

The term "therapeutic agent", as it is used herein, refers to any compound or component that has therapeutic activity. In particular, it refers to agents commonly used to treat infections in a localized manner, such as antimicrobial or antiseptic agents. As will be understood by one skilled in the art, antimicrobial agents include antibacterial agents, antifungal agents, antiviral agents, antiseptic agents, etc.

The term "antimicrobial agent", "antiseptic agent", "antibacterial agent", "antifungal agent", "antiviral agent" refer to corresponding antimicrobial, antiseptic, antibacterial, antifungal, or antiviral compounds or components commonly known to a person skilled in the art.

The expression "in contact with", in relation to the therapeutic agent on the surface of the nanoparticle of the invention, refers to the fact that said agent is retained on the surface of the nanoparticle. Said retention can be the result of an attachment (physical or chemical) or simply of retention by a physical barrier that prevents the agent from being released from the surface of the nanoparticle. If the retention results from an attachment, it would preferably be a weak attachment, preferably a weak chemical bond, between the therapeutic agent and the surface of the nanoparticle. In a particular embodiment, weak chemical bonds referred to in the invention refer to ionic bonds, *Van der Waals* bonds, or hydrogen bonds, commonly known to a person skilled in the art. If it is a physical barrier, it would be, for example, an element comprised in the nanodevice that, due to its three-dimensional structure and arrangement, prevents the agent from being released from, or lose contact with, the surface of the nanoparticle. In a particular embodiment, the retention of the therapeutic agent on the surface of the nanoparticle (either resulting from an attachment or simply a physical barrier to prevent release from the surface of the nanoparticle), is reversible in a specific environment, either because the weak bond is broken under certain conditions in said environment, or because the element comprised in the nanodevice that acts as a physical barrier loses its three-dimensional structure and/or arrangement with respect to said therapeutic agent and allows the release thereof under specific conditions of said nanodevice.

In a particular embodiment, the therapeutic agent in contact with the surface of the nanoparticle is attached to the surface of the nanoparticle, or comprised and retained in the pores of the nanoparticle, preferably in a pore of the nanoparticle. As will be understood by one skilled in the art, if the therapeutic agent is attached to the surface of the nanoparticle, this attachment is preferably weak. In a preferred embodiment, said attachment is an ionic type chemical attachment, *Van der Waals* bonds, or hydrogen bonds, preferably an ionic type chemical attachment. If it is comprised in the pores of the nanoparticle, preferably in a pore of the nanoparticle, it is retained by a physical barrier, preferably by another component comprised in the nanoparticle of the invention the three-dimensional structure and/or arrangement of which with respect to the therapeutic agent prevents the therapeutic agent from being released from, or coming out of, the pore of the nanoparticle. In a particular embodiment, said component is the molecular drill of the invention. In a preferred embodiment, the component that retains the agent comprised in the pores/pore of the nanoparticle completely or partially obstructs the opening of the pore in which the therapeutic agent is comprised. In a preferred embodiment, the component that retains the therapeutic agent in the pores/pore of the nanoparticle is the molecular drill of the invention, preferably the molecular complex of the molecular drill of the invention.

The term "pore", as it is used herein, refers to a cavity, gap, or hole in the nanoparticle of the invention, preferably on its surface, which is in contact with the external environment of the nanoparticle by means of a small opening (pore diameter) on the surface of the nanoparticle. Pores can be classified as micropores when the diameter of the pore opening hole is less than 2 nm, macropores when the diameter of the pore opening hole is more than 50 nm, or mesoporous when the diameter of the pore opening hole is 2-50 nm.

Nanoparticle of the Invention

The nanoparticle of the invention refers to the nanoparticle comprised in the nanodevice of the invention.

In a particular embodiment, the nanoparticle of the invention is of the organic or inorganic type.

The term "organic nanoparticle", as it is used herein, refers to a nanoparticle, as defined above, wherein the components forming same can be obtained from or generated by a living organism. Said organic compounds are generally polymers, repeating structures, lipid bilayers, or derivatives of any of these. In a particular embodiment, organic nanoparticles form polymer-like structures, liposome-like structures, micelle, dendrimer, or a protocell. Preferably, said organic particles are of the PGLA, PANAM, or chitosan type, among others.

The term "inorganic nanoparticle", as it is used herein, refers to a nanoparticle formed by metals and inert materials such as titanium dioxide, hydroxyapatite, or silica. Inorganic nanoparticles can be porous or nonporous, wherein the term "porous" refers to the fact that the nanoparticle comprises pores as defined above. As will be understood by one skilled in the art, inorganic nanoparticles can be microporous if they comprise micropores, mesoporous if they comprise mesopores, or macroporous if they comprise macropores, wherein micropore, mesopore, and macropore refer to the terms indicated in the definition of a pore above. Non-limiting examples of porous inorganic nanoparticles include mesoporous silica nanoparticles from the MCM (Mobil Composition Matter) family or M41S of different types: MCM-41 (two-dimensional, hexagonal), MCM-48 (three-dimensional, cubic), MCM-50 (laminar phase), from the SBA (Santa Barbara Amorphous) family of different types SBA-1, SBA-2, SBA-3, SBA-6, SBA-8, SBA-11, SBA-12, SBA-14, SBA-15, SBA-16; from the FSM family, such as FSM-16, HMS, MSU such as MSU-1, MSU-2, MSU-3, MSU-V, or KIT-1. As will be understood by one skilled in the art, the shape of inorganic nanoparticles is generally spherical, hexagonal, or elongated, preferably spherical.

In a particular embodiment, the nanoparticle of the invention is a porous inorganic nanoparticle selected from the list consisting of MCM-41, MCM-48, MCM-50, and SBA, wherein the SBA nanoparticle is preferably SBA-15.

In another particular embodiment, the nanoparticle of the invention is an organic nanoparticle selected from the list consisting of a polymeric nanoparticle, a liposome, a micelle, a dendrimer, or a protocell.

Molecular Drill of the Invention

The molecular drill of the invention refers to the molecular drill comprised in the nanodevice of the invention.

In one embodiment of the invention, the molecular drill comprises at least one compound with catalytic activity attached to a molecular complex that is attached to the surface of the nanoparticle.

The term "compound with catalytic activity", as it is used herein, refers to a compound capable of accelerating or promoting the breakage of a specific organic structure, in particular, an organic structure with the composition of a biofilm generated by at least one microbe, preferably by bacteria and fungi. Non-limiting examples of compounds with catalytic activity include enzymes, mucolytic agents, lipopeptides, chitosan, cis-2-decanoic acid (C2DA), nitric oxide, rhamnolipids, or cerium IV (see, for example, compounds that promote the breakage of biofilms in Pinto R.M. et al., Frontiers in Microbiology, 2020, 11: 952, doi: 10.3389/fmicb.2020.00952).

The term "molecular complex" has been defined above. In the context of the molecular complex that is part of the molecular drill of the invention, the molecular complex comprises a first molecular unit that retains the therapeutic agents comprised on the surface of the nanoparticle of the invention (due, for example, to its large-volume three-dimensional structure and to the fact that its arrangement on the surface of the nanoparticle completely or partially obstructs the pore/pores of the nanoparticle comprising/the therapeutic agent), and a second molecular unit the electrical charge of which, and/or attachment with the first molecular unit is altered by changes in the pH of the medium, preferably by reductions in the pH of the medium. Both molecular units are attached by weak chemical bonds, by means of intermolecular forces, preferably of the *Van der Waals* and/or hydrophobic interactions type. As will be understood by one skilled in the art, said bonds can be broken in response to changes in conditions of the medium, such as, for example, the pH changes mentioned above, and/or in response to changes in the electrical charge of any of the components thereof. By altering or breaking the bond between the first and second molecular units of the molecular complex, the arrangement of the first molecular unit changes with respect to the therapeutic agent and/or the pore of the nanoparticle in which it is located. In a particular embodiment, said changing arrangement consists of the obstruction of the pore/pores of the nanoparticle in which the therapeutic agent/agents of the invention are located. In this way, the first molecular unit and the molecular drill together may no longer retain the therapeutic agent on the surface of the nanoparticle.

In a particular embodiment, the arrangement of the first molecular unit of the molecular complex that is part of the molecular drill, and therefore of the molecular drill, that retains the therapeutic agent of the invention on the surface of the nanoparticle is an arrangement consisting of the obstruction of the pore/pores of the nanoparticle in which the therapeutic agent/agents of the invention are located. In a preferred embodiment, it consists of the obstruction of at least one pore of the nanoparticle in which the at least one therapeutic agent of the invention is located, preferably in part due to the high volume of its quaternary structure. Therefore, as will be understood by one skilled in the art, the change in the arrangement of the first molecular unit referred to above, and therefore of the molecular drill of the invention, that allows the release of the therapeutic agent of the invention, preferably consists of said molecular unit and molecular drill no longer obstructing the at least one pore of the nanoparticle comprising the at least one therapeutic agent of the invention.

Therefore, in a particular embodiment, the molecular complex that is part of the molecular drill is characterized in that, in response to changes in the pH of the medium, preferably in response to reductions in the pH of the medium, allows the release of the therapeutic agent in contact with the surface of the nanoparticle.

In a particular embodiment, the pH at which the molecular complex of the molecular drill of the invention allows the release of the therapeutic agent from the surface of the nanoparticle of the invention is less than 7; 6.5; 6; 5.5; 5; 4.5; 4, 3.5; 3; 2.5; 2; 1.5; 1, preferably less than 5. In another particular embodiment, the pH at which the molecular complex of the molecular drill of the invention allows the release of the therapeutic agent from the surface of the nanoparticle of the invention is about 5.5; 5; 4.5; 4; 3.5; 3; 2.5; 2; 1.5; 1, preferably around 5.

In another particular embodiment, the second molecular unit of the molecular complex that is part of the molecular drill deprotonates at a pH equal to or less than 7; 6.5; 6; 5.5; 5; 4.5; 4, 3.5; 3; 2.5; 2; 1.5; 1, preferably less than 5. In another particular embodiment, the second molecular unit of the molecular complex that is part of the molecular drill deprotonates at a pH of about 7; 6.5; 6; 5.5; 5; 4.5; 4, 3.5; 3; 2.5; 2; 1.5; 1, preferably about 5.

The term "deprotonate" or "deprotonation" refers to the transfer of a hydrogen cation (H+) by a molecule, forming the respective conjugated base. The relative ease with which a molecule can donate an H+ cation is measured by its pKa value. A low pKa value indicates that the compound is acidic and will readily transfer an H+ cation to a base. As will be understood by one skilled in the art, in the context of the invention, the deprotonation of the second molecular unit of the molecular complex of the molecular drill of the invention (and therefore the change in electrical charge thereof) gives rise to an alteration in the attachment between the molecular units of the molecular complex that are part of the molecular drill, and therefore to a change in arrangement of the first molecular unit of the complex and the molecular drill as a whole with respect to the therapeutic agent of the nanoparticle, and/or the pore in which it is located, and the non-retainment of the therapeutic agent on the surface of the nanoparticle.

In a particular embodiment, the attachment between the molecular drill and the surface of the nanoparticle is by means of a chemical bond, as defined in the definition of attachment above, preferably a covalent bond. In another particular embodiment, the attachment between the molecular drill and the surface of the nanoparticle is by means of a chemical bond between the second molecular unit of the molecular complex that is part of the molecular drill of the invention, as defined above, and the surface of the nanoparticle. Therefore, in a preferred embodiment, the attachment between the molecular drill and the surface of the nanoparticle is by means of a covalent bond between the second molecular unit of the molecular complex that is part of the molecular drill of the invention, as defined above, and the surface of the nanoparticle.

In a particular embodiment, the nanoparticle is made of silica, or the outer surface of the nanoparticle is made of silica, and the bond between the molecular drill and the surface of the nanoparticle, as described in the embodiments right above, is carried out between a silane molecule on the surface thereof and the corresponding molecule of the molecular complex that is part of the molecular drill of the invention, preferably the second molecular unit of said molecular complex. In a preferred embodiment, the bond between the silane molecule and the molecule of the molecular complex mentioned above is a covalent bond.

In a particular embodiment, the nanoparticle of the invention comprises several molecular drills attached to its surface. In a particular embodiment, the drills attached to the surface of the nanoparticle are attached at different points on the nanoparticle, preferably at different points distributed homogeneously on the surface of the nanoparticle. In a preferred embodiment, the surface of the nanoparticle is surrounded by molecular drills. In a particular embodiment, the nanoparticle comprises several molecular drills attached to its surface, covering 80%, 75%, 70%, 65%, 50%, 45%, 40%, 30%, 20%, 10% of the surface of the nanoparticle. In another preferred embodiment, the nanoparticle comprises several molecular drills attached to its surface, covering 80%, 75%, 70%, 65%, 50%, 45%, 40%, 30%, 20%, 10% of the surface of the nanoparticle that does not comprise the self-propulsion system of the invention. In another particular embodiment, a 100%, 90%, 80%, 75%, 70%, 65%, 50%, 45%, 40%, 30%, 20%, 10% of the pores of the nanoparticle are covered by the molecular drills of the invention, preferably by the first molecular unit of the molecular drills of the invention.

In another preferred embodiment, the molecular drill of the invention is attached to the self-propulsion system of the invention by means of a covalent bond through a thiol (SH) group.

Particular Embodiments of the Molecular Complex of the Molecular Drill of the Invention

In a particular embodiment, the first molecular unit of the molecular complex that is part of the molecular drill as defined above is a cyclodextrin. In another particular embodiment, the second molecular unit of the molecular complex that is part of the molecular drill as defined above is a hydrophobic molecule, preferably an imidazole derivative, or an aniline.

Therefore, in a preferred embodiment, the first molecular unit of the molecular complex that is part of the molecular drill as defined above is a cyclodextrin and the second molecular unit of said molecular complex is a hydrophobic molecule, preferably an imidazole derivative, or an aniline.

The term "cyclodextrin", as it is used herein, is a family of compounds formed by monosaccharide molecules attached in the form of a ring (cyclic oligosaccharides), produced from starch by enzymatic conversion. Typically cyclodextrins contain a number of glucose monomers ranging from six to eight units in a ring, creating a cone shape. More specifically, cyclodextrins are made up of 6-8 glucopyranoside units and can be topologically represented as toroids, with the largest and smallest openings exposed to the secondary and primary hydroxyl groups, respectively. Due to this arrangement, the interior of the toroids is not hydrophobic, but it is considerably less hydrophilic than the aqueous medium and therefore capable of housing other hydrophobic molecules. By contrast, the exterior is sufficiently hydrophilic to impart to cyclodextrins their solubility in water.

The formation of inclusion compounds greatly modifies the physical and chemical properties of the guest molecule, primarily in terms of its solubility in water. Under specific conditions, inclusion compounds can release the guest molecule. Said conditions may consist of changes in the pH of the medium, which leads to the loss of hydrogen or ionic bonds between the host and the guest molecules. Alternative means for disrupting the complexes may also take advantage of the actions of enzymes capable of cleaving $\alpha$-1,4 bonds between glucose monomers.

Cyclodextrin is named with a different Greek letter according to the number of glucose units forming same:

- $\alpha$-Cyclodextrin: n = 6 glucose molecules (Diameter/-Height of cavity: 4.7..5.3/7.9A),

- $\beta$-Cyclodextrin: n = 7 glucose molecules (Diameter/-Height of cavity: 6.0..6.5/7.9A),

- $\gamma$-Cyclodextrin: n = 8 glucose molecules (Diameter/-Height of cavity: 7.5..8.3/7.9A),

- $\delta$-Cyclodextrin: n = 9 glucose molecules.

In a particular embodiment, the cyclodextrin of the molecular complex that is part of the molecular drill of the invention is an $\alpha$-cyclodextrin, $\beta$-cyclodextrin, or $\Upsilon$-cyclodextrin, preferably a $\beta$-cyclodextrin.

In a preferred embodiment, the first molecular unit of the molecular complex that is part of the molecular drill as defined above is a cyclodextrin, characterized in that a hydrophobic molecule is housed in the non-hydrophilic interior of the cyclodextrin toroid. Therefore, in a particular embodiment, the molecular complex of the molecular drill comprises an inclusion complex of cyclodextrin, characterized in that a hydrophobic molecule is housed in the non-hydrophilic interior of the cyclodextrin toroid. In a particular embodiment, the hydrophobic molecule is imidazole, an imidazole derivative, or an aniline. In a preferred embodiment, the hydrophobic molecule is imidazole, an imidazole derivative, or an aniline that deprotonates at a pH equal to or less than 5.5, preferably equal to or less than 5. In a particular embodiment, the hydrophobic molecule is the second molecular unit of the molecular complex that is part of the molecular drill as defined above.

The term "imidazole", as it is used herein, refers to an intermediate of histidine biosynthesis that is formed from imidazole glycerol phosphate with loss of water. Its IUPAC name is 1H-imidazole. In a particular embodiment, imidazole is considered a strong base and a weak acid that deprotonates at a pH equal to or less than 5. The term "imidazole derivative", as it is used herein, refers to compounds comprising an imidazole group. Non-limiting examples of imidazole derivatives include benzimidazole, bifonazole, ketoconazole, tioconazole, miconazole, itraconazole.

In a particular embodiment, the imidazole derivative is benzimidazole, bifonazole, ketoconazole, tioconazole, miconazole, itraconazole, or combinations thereof.

The term "aniline", as it is used herein, refers to the compound with the IUPAC name phenylamine. In a particular embodiment, it deprotonates at a pH equal to or less than 5.

In a particular embodiment, the first molecular unit of the complex that is part of the molecular drill of the invention is an $\alpha$-cyclodextrin, $\Upsilon$-cyclodextrin, or a cucurbituril, and the second molecular unit of the complex is imidazole, an imidazole derivative, an aniline, A polyethyleneimine (PEI)-type linear polymer, or p-anisidine. In a particular embodiment, the first molecular unit of the complex is a cucurbituril and the second molecular unit is an aniline or a p-anisidine. Similar to the case where the complex comprises a cyclodextrin and a hydrophobic molecule therein, at neutral pH the complex formed by cucurbituril and aniline or p-anisidine is stable and uncouples at acidic pH, in particular at a pH of about 5.

In another particular embodiment, the first molecular unit of the molecular complex forming the molecular drill of the invention is a cucurbituril and the second molecular unit of the complex is a polymer with amino groups. In this case, the complex is stable at neutral pH and destabilizes at basic pH, in particular at a pH of about 10. Preferably, the amino groups of the aliphatic chain or the polymer must be protonated, and as the pH increases, said charge disappears displacing the cucurbituril.

The term "cucurbituril", as it is used herein, refers to macrocyclic molecules formed by of glycoluril ($=C_4H_2N_4O_2=$) attached by methylene bridges ($-CH_2-$). The location of oxygen atoms and their spatial arrangement results in these molecules having a partially closed cavity.

In another embodiment of the invention, the second molecular unit of the molecular complex that is part of the molecular drill of the invention is a chelating subunit, such as an iminodiacetate, phenanthroline, or N-(3-trimethoxysilpropyl)ethylenediamine. As will be understood by one skilled in the art, said molecular unit is capable of coordinating with a first molecular unit as defined above comprising cations (for example, $Cu^{2+}$, $Co^{2+}$, $Ni^{2+}$, and $Ca^{2+}$) at neutral pH, forming complexes that block the surface of the nanoparticles of the invention, preventing the release of the therapeutic agents. In a particular embodiment, molecular complexes comprising a chelating subunit and a group comprising cations are called "M2+-Ligand" type molecular complexes.

Under low pH conditions, for example, less than 5, protonation of the chelating subunit occurs, and therefore the disassembly of the M2+-ligand complex. Said modification allows the release of the therapeutic agent comprised on the surface of the nanoparticles of the invention.

In a particular embodiment, the molecular complex that is part of the molecular drill of the invention is an assembly of molecular units of the same type. In a particular embodiment, it is an assembly of chitosan units. In another particular embodiment, the molecular complex that is part of the molecular drill of the invention is an assembly of polyamines.

The term "chitosan", as it is used herein, refers to a biopolymer of aminopolysaccharides, made up of randomly distributed units of β-(1-4) D-glucosamine (deacetylated units) and N-acetyl-D-glucosamine (acetylated unit). Chitosan is produced commercially by means of the partial deacetylation of chitin, which is a structural element in the exoskeleton of crustaceans (crabs, prawns, lobsters, etc.), as well as insects. Due to the presence of amino groups in its composition, chitosan is sensitive to pH changes. At neutral pH, chitosan remains in its unprotonated form and changes conformation at acidic pH (about 6 or 4).

The term "polyamine", as it is used herein, refers to molecules of a polycationic nature present in plants, animals, and microorganisms. The most eminent polyamines in plants are the diamine putrescine, the triamine spermidine, and the tetraamine spermine. At acidic pH (about 2-3.7), the amino groups of polyamines are generally protonated and they generally deprotonoate at higher pH (5.5-7), losing their stable or rigid conformation.

As will be understood by one skilled in the art, if the molecular complex that is part of the molecular drill is formed by an assembly of molecular units of the same type, the attachment between the molecular drill and the surface of the nanoparticle takes place between at least one of the molecular units forming said assembly and the surface of the nanoparticle. Said attachment is preferably a chemical bond as defined in the definition of "attachment" above, preferably a covalent bond.

Chitosan can be attached to the surface of nanoparticles by different pathways, such as, for example, modifying with silanes (for example, (3-glyciloloxypropyl)trimethoxysilane), which facilitates the anchoring thereof to the external surface of nanoparticles, particularly to nanoparticles with an external surface made of silica. In a particular embodiment, the attachment between chitosan, or at least one chitosan of the molecular complex, and the surface of the nanoparticles, particularly the attachment generated by the reaction with silane groups, is a covalent bond. Once attached to the surface of the nanoparticles of the invention in its unprotonated form, the assembly of chitosan molecules retains the therapeutic agents on the surface of the nanoparticles of the invention due to its large-volume tertiary structure and/or arrangement on the surface of the nanoparticles, but when pH decreases, it becomes protonated, changes conformation, and allows the release of the therapeutic agents comprised on the surface of the nanoparticle.

The attachment of polyamines to the surface of the nanoparticles of the invention, particularly to nanoparticles with an external surface made of silica, is generated by reacting with different types of silanes (for example, [2-(2-aminoethylamino)ethylamino]propylmethoxysilane). In a particular embodiment, the attachment between the polyamines and the surface of the nanoparticles, particularly the attachment generated by the reaction with silane groups, is a covalent bond. At acidic pH (about 2-3.7), they have a stable or rigid conformation that, due to their three-dimensional structure and arrangement on the surface of the nanoparticles, retain therapeutic agents on the surface of the nanoparticle, and at higher pH (5.5-7), it deprotonates, loses its stable configuration, and allows the release of therapeutic agents from the nanoparticles of the invention.

In a particular embodiment, molecular complexes that do not comprise first and second molecular units, but rather an assembly of molecules, such as those formed by chitosan and polyamides, the tertiary structure, and/or the arrangement of the molecular complex, and therefore of the molecular drill, retaining the therapeutic agents on the surface of the nanoparticle, preferably consists of the fact that they obstruct the at least one pore of the nanoparticle comprising the at least one therapeutic agent of the invention. As will be understood by one skilled in the art, the change in the tertiary structure and/or in the arrangement of the molecular complex, and therefore of the molecular drill, that allows the release of the therapeutic agent of the invention, preferably comprises the fact that the tertiary structure of said assembly of chitosan or polyamine and/or the arrangement thereof no longer obstructs the at least one pore of the nanoparticle comprising the at least one therapeutic agent of the invention.

Particular Embodiments of the Compound with Catalytic Activity of the Molecular Drill of the Invention

As indicated above, in addition to the molecular complex, the molecular drill comprises a compound with catalytic activity, as defined above. In a particular embodiment, the attachment between the compound with catalytic activity and the molecular complex that is part of the molecular drill of the invention is a chemical bond as described in the definition of "attachment" above, preferably a covalent bond.

The term "biofilm", as it is used herein, and particularly in the definition of compound with catalytic activity above, refers to a matrix of extracellular polymeric substances (EPS) that surrounds one or more colonies of infectious agents, i.e., microbial agents, particularly bacteria, but also fungi. The matrix or biofilm is responsible for intercellular interactions and for protecting microbial cells from hostile environments. Therefore, said matrices or biofilms increase the tolerance and resistance of bacteria to antibiotics in comparison with cells in planktonic state. Biofilms are made up mainly of polysaccharides, lipids, proteins, and extracellular DNA. The relative amount of each of these compounds can vary from one biofilm to another. For example, the biofilms generated by *Pseudomonas aeruginosa (P. aeruginosa)* comprise a large amount of extracellular DNA, while those generated by *Staphylococcus epidermidis (S. epidermidis)* comprise a small amount of extracellular DNA.

Biofilm formation can be divided into 3 main stages: adhesion, maturation, and dispersion. Biofilms are formed once microbial cells, particularly bacterial and fungal cells, adhere to a substrate or to other cells embedded in an extracellular polymeric matrix. For example, when a medical device is introduced into an animal body, protein matrices that adhere to the surface of the device are formed. Microbial cells, such as bacterial cells, can recognize the proteins of said matrix, adhere thereto, and promote colonization. At this point, the biofilm grows due to the formation of the EPS matrix around the microbial cells until reaching a maturation phase, adopting a three-dimensional structure. In response to a stimulus from the medium, some cells or groups of cells can detach from the biofilm and promote dissemination and colonization in other areas of the host. For more details see Pinto R.M. et al., Frontiers in Microbiology, 2020, 11: 952, doi: 10.3389/fmicb.2020.00952).

Methods to determine the presence of a biofilm formed by certain organisms are commonly known by one skilled in the art, as well as the identification of the microorganisms generating same. Non-limiting examples of said methods include those described in Metcalf et al., J Wound Care, 2014, 23(3): 137-42, Torregrosa Jere L. y Verdú Soriano J., "Diagnostic methods for the identification of biofilm in chronic wounds. Systemic Review", Univ. Alicante, 2015. Methods to determine the microorganisms that generate a biofilm are described in detail in Verdú Soriano J., "Diagnostic methods for the identification of biofilm in chronic wounds. Systemic Review", Univ. Alicante, 2015. As will be understood by one skilled in the art, to identify said microorganisms, the method for identifying the presence of a biofilm described above can be combined with the identification of microorganisms found in a sample comprising said biofilm, wherein the identification of said microorganisms can be performed by methods commonly known by one skilled in the art such as those also described in Infectious Diseases and Clinical Microbiology, 2011, Vol. 29. No. 8., DOI: 10.1016/j.eimc.2011.03.012), by means of phenotypic and/or genetic analysis using different markers such as 16S rRNA, fluorescent *in situ* hybridization (FISH), pyrosequencing, or quantitative PCR (Q-PCR).

In a preferred embodiment, microorganisms, bacteria, and/or fungi referred to in the present invention form biofilms as have been defined. Microorganisms, bacteria, and/or fungi referred to in the present invention are defined and described in more detail in the third aspect of the invention, and apply to the first and second aspects of the invention.

In a particular embodiment, the at least one compound with catalytic activity attached to the molecular complex of the molecular drill of the invention is selected from the list consisting of an enzyme, a mucolytic agent, a lipopeptide, chitosan, cis-2-decanoic acid (C2DA), nitric oxide, rhamnolipids, cerium IV, and combinations thereof.

The term "enzyme" refers to the compounds commonly known by the term "enzyme", generally of protein origin or RNA origin (ribozymes), that change the rate of a particular reaction without affecting the equilibrium thereof, acting on specific substrates of each enzyme that are converted into products. Non-limiting examples of enzymes in the context of the invention include proteases, DNAses, glycosidases, amylases, cellulases, dispersin B, pancreatin, or alginate lyase.

In a particular embodiment, the at least one compound with catalytic activity is an enzyme which is selected from the list consisting of a protease, DNAse, glycosidase, amylase, cellulase, dispersin B, pancreatin, alginate lyase, and combinations thereof. In a particular embodiment, the at least one compound with catalytic activity comprises an enzyme which is selected from the list consisting of a protease, DNAse, glycosidase, amylase, cellulase, dispersin B, pancreatin, alginate lyase, and combinations thereof.

As understood by one skilled in the art, proteases are enzymes that break peptide bonds in proteins. Non-limiting examples of proteases in the context of the invention include ficin, proteinase K, trypsin, lysostaphin, bromelain, lysostaphin, papain, or peptidase M16. Therefore, in a particular embodiment, the protease is selected from the list consisting of ficin, proteinase K, trypsin, lysostaphin, bromelain, lysostaphin, papain, peptidase M16, and combinations thereof. In another particular embodiment, the at least one compound with catalytic activity comprises a protease which is selected from the list consisting of ficin, proteinase K, trypsin, lysostaphin, bromelain, lysostaphin, papain, peptidase M16, and combinations thereof. In a preferred embodiment, the protease is ficin. In a particular embodiment, the at least one compound with catalytic activity comprises ficin.

On the other hand, DNAses are enzymes that catalyze the hydrolytic breakage of phosphodiester bonds in DNA. Non-limiting examples of DNAses in the context of the invention include DNAse I or NucB. Therefore, in a particular embodiment, the DNAse is selected from the list consisting of DNAse I, NucB, and combinations thereof. In another particular embodiment, the at least one compound with catalytic activity comprises a DNAse which is selected from the list consisting of DNAse I, NucB, and combinations thereof.

Glycosidases are enzymes that catalyze the breakage of glycosidic bonds. Non-limiting examples of glycosidases in the context of the invention include glycoside hydrolases PelAh and PsIGh (Baker et al. 2016, Sci. Adv. 2:e1501632; doi: 10.1126/sciadv.1501632). Therefore, in a particular embodiment, the glycosidase enzyme is selected from the list consisting of glycoside hydrolase PelAh, PsIGh, and combinations thereof. In another particular embodiment, the at least one compound with catalytic activity comprises a glycosidase enzyme which is selected from the list consisting of the glycoside hydrolase PelAh, PsIGh, and combinations thereof.

Amylases are enzymes that catalyze the hydrolysis of the 1-4 bonds between glucose units. Non-limiting examples of amylases in the context of the invention include α-amylase. In a particular embodiment, the amylase is α-amylase. In another particular embodiment, the at least one compound with catalytic activity comprises α-amylase.

Cellulases are enzymes specializing in catalyzing cellulolysis, in other words, in breaking down cellulose and other related polysaccharides, into multiple glucose monomers. Non-limiting examples of cellulases include endocellulases (EC 3.2.1.4), exocellulases (EC 3.2.1.91, also referred to as cellobiohydrolases), cellobiases (EC3.2.1.21, also referred to as β-glycosidases), oxidative cellulases that depolymerize cellulose by means of free radical reactions (for example, cellobiose dehydrogenase), or cellulose phosphorylases that depolymerize cellulose using phosphate groups instead of water. Therefore, in a particular embodiment, cellulase is selected from the list consisting of endocellulase, exocellulase, cellobiase, oxidative cellulase, cellulose phosphorylase, and combinations thereof. In another particular embodiment, the at least one compound with catalytic activity comprises a cellulase which is selected from the list consisting of endocellulase, exocellulase, cellobiase, oxidative cellulase, cellulose phosphorylase, and combinations thereof.

The term "mucolytic agent" refers to substances that have the ability to destroy different chemical-physical structures of abnormal bronchial secretion, or phlegm, achieving a decrease in viscosity. Non-limiting examples of mucolytic agents include surface active agents such as propylene glycol (IUPAC name propane-1,2-diol) or tyloxapol (IUPAC name formaldehyde; oxirane;4-(2,4,4-trimethylpentan-2-yl)phenol), amino acid derivatives such as carboxymethylcysteine (IUPAC name 2-amino-3-(carboxymethylsulfanyl)propanoic acid), N-acetylcysteine (NAC, IUPAC name (R)-2-acetamido-3-sulfanylpropanoic acid) or MESNA (IUPAC name sodium; 2-sulfanylethanesulfonate), enzymes such as trypsin or chymotrypsin, synthetic derivatives such as bromhexine or ambroxol (IUPAC name trans-4-(2-amino-3,5-dibromobenzylamino)-cyclohexanol). Therefore, in a particular embodiment, the mucolytic agent is selected from the list consisting of ambroxol, N-acetylcysteine (NAC), propylene glycol, tyloxapol, carboxymethylcysteine, MESNA, bromhexine, and combinations thereof. In another particular embodiment, the at least one compound with catalytic activity comprises a mucolytic agent which is selected from the list consisting of ambroxol, N-acetylcysteine (NAC), propylene glycol, tyloxapol, carboxymethylcysteine, MESNA, bromhexine, and combinations thereof.

The term "lipopeptide", as it is used herein, refers to molecules consisting of a lipid connected to a peptide. Some lipopeptides are known for their antibacterial or antifungal activity. Non-limiting examples of lipopeptides in the context of the invention include caspofungin acetate. Therefore, in a particular embodiment, the lipopeptide is caspofungin acetate. In another particular embodiment, the at least one compound with catalytic activity comprises caspofungin acetate.

Self-propulsion System of the Invention

The self-propulsion system of the invention refers to that the system comprised in the nanodevice of the invention.

As indicated above, the nanodevice further comprises a self-propulsion system defined above.

In one embodiment, the self-propulsion system of the nanodevice of the invention comprises a metal which is selected from the list consisting of platinum (Pt), silver (Ag), iridium (Ir), nickel (Ni), zinc (Zn), and combinations thereof. In a preferred embodiment, the metal is platinum (Pt). In another embodiment, the self-propulsion system of the nanodevice of the invention comprises the mineral $MgO_2$. In another embodiment, the self-propulsion system of the nanodevice of the invention comprises an enzyme which is selected from the list consisting of urease, glucose oxidase, and combinations thereof. In another embodiment, the self-propulsion system of the nanodevice of the invention comprises a metal which is selected from the list consisting of platinum (Pt), silver (Ag), iridium (Ir), nickel (Ni), zinc (Zn), magnesium (Mg), and combinations thereof, preferably Pt, the mineral $MnO_2$, and/or an enzyme selected from the list consisting of urease, glucose oxidase, and combinations thereof. In a preferred embodiment, the self-propulsion system of the nanodevice of the invention comprises the metal Pt.

As indicated above, the self-propulsion system is attached to the surface of the nanoparticle of the invention by means of a chemical bond or a physical attachment. In the case of an enzyme, the attachment is preferably a chemical bond as described in the definition of attachment above. In a preferred embodiment, if the self-propulsion system comprises an enzyme, it is attached to the surface of the nanoparticle by means of a chemical bond, preferably a covalent bond.

In said preferred embodiment, the bond would be an amide-type covalent bond. Said bond is established between a silane and the enzyme.

In another particular embodiment, if the system comprises a metal or a mineral, the attachment is preferably physical. In a preferred embodiment, said physical attachment is the result of the deposition of all or part of the metal or mineral of the self-propulsion system. Methods for attaching molten material to the nanoparticle are commonly known to one skilled in the art.

For example, once the metal or mineral is melted, it is deposited by means of the vaporization of its atoms (in gaseous state), after being bombarded with energetic ions (plasma), on a region of the surface of the nanoparticle, preferably at a specific point of the surface of the nanoparticle.

In a particular embodiment, the self-propulsion system comprises a metal or mineral, wherein the metal or mineral is in the form of a nanoparticle or a layer surrounding part of the surface of the nanoparticle. In a particular embodiment, the part of the surface of the nanoparticle occupied by the self-propulsion system is less than 75%, 70%, 65%, 60%, 55%, 50%, 45%, 40%, 35%, 30%, 25%, 20%, 15%, 12%, 10%, 9%, 8%, 7%, 6%, 5%, 4%, 3%, 2%, 1%, preferably less than 25%.

In a particular embodiment, the self-propulsion system comprises a metal or mineral in the form of a nanoparticle as defined above. In a particular embodiment, the self-propulsion system comprises a metal or mineral in the form of a nanoparticle and the physical attachment to the surface of the nanoparticle is the result of the deposition of 50%, 40%, 30%, 25%, 20%, 18%, 15%, 12%, 10%, 7%, 5%, 4%, 3%, 2%, 1% of the material forming the nanoparticle of the self-propulsion system. In another particular embodiment, the physical attachment is the result of the deposition of 50%, 40%, 30%, 25%, 20%, 18%, 15%, 12%, 10%, 7%, 5%, 4%, 3%, 2%, 1% of the metal forming the nanoparticle of the self-propulsion system. In another particular embodiment, the physical attachment is the result of the deposition of 50%, 40%, 30%, 25%, 20%, 18%, 15%, 12%, 10%, 7%, 5%, 4%, 3%, 2%, 1% of the mineral forming the nanoparticle of the self-propulsion system. In another particular embodiment, the physical attachment is the result of the deposition of 50%, 40%, 30%, 25%, 20%, 18%, 15%, 12%, 10%, 7%, 5%, 4%, 3%, 2%, 1% of the volume of the nanoparticle of the self-propulsion system. In another particular embodiment, the physical attachment is the result of the deposition of 50%, 40%, 30%, 25%, 20%, 18%, 15%, 12%, 10%, 7%, 5%, 4%, 3%, 2%, 1% of the volume of the metal nanoparticle of the self-propulsion system. In another particular embodiment, the physical attachment is the result of the deposition of 50%, 40%, 30%, 25%, 20%, 18%, 15%, 12%, 10%, 7%, 5%, 4%, 3%, 2%, 1% of the volume of the mineral nanoparticle of the self-propulsion system.

In another particular embodiment, the self-propulsion system comprises a metal or mineral layer that surrounds part of the surface of the nanoparticle of the invention. In a particular embodiment, the metal or mineral layer of the self-propulsion system surrounds the surface of the nanoparticle occupied by said self-propulsion system. In another particular embodiment, the metal layer of the self-propulsion system surrounds the surface of the nanoparticle occupied by said self-propulsion system. In another particular embodiment, the mineral layer of the self-propulsion system surrounds the surface of the nanoparticle occupied by said self-propulsion system. In a preferred embodiment, the surface of the nanoparticle that is surrounded by the metal layer of the self-propulsion system is selected from the percentages of surface of the nanoparticle occupied by the self-propulsion system indicated in the embodiment above. In another preferred embodiment, the surface of the nanoparticle that is surrounded by the mineral layer of the self-propulsion system is selected from the percentages of surface of the nanoparticle occupied by the self-propulsion system indicated in the embodiment above.

In a particular embodiment, the self-propulsion system comprises a metal in the form of a layer and the physical attachment to the surface of the nanoparticle is the result of the deposition of 100%, 90%, 85%, 80%, 75%, 70%, 65%, 60%, 65%, 50%, 45%, 40%, 35%, 30%, 25%, 20%, 15%, 12%, 10%, 9%, 8%, 7%, 6%, 5% - 4%, 3%, 2%, 1%, preferably 100%, of the volume of the metal layer of the self-propulsion system. In another particular embodiment, the self-propulsion system comprises a mineral in the form of a layer and the physical attachment to the surface of the nanoparticle is the result of the deposition of 100%, 90%, 85%, 80%, 75%, 70%, 65%, 60%, 65%, 50%, 45%, 40%, 35%, 30%, 25%, 20%, 15%, 12%, 10%, 9%, 8%, 7%, 6%, 5% - 4%, 3%, 2%, 1%, preferably 100%, of the volume of the mineral layer of the self-propulsion system.

In a particular embodiment, the self-propulsion system comprises an enzyme and a metal or mineral, wherein the enzyme is attached to the metal or mineral as defined above and described in the particular embodiments above.

In a particular embodiment, the self-propulsion system comprises an enzyme and a metal nanoparticle, wherein the enzyme is attached to the metal nanoparticle as defined above and described in the particular embodiments above. In another particular embodiment, the self-propulsion system comprises an enzyme and a mineral nanoparticle, wherein the enzyme is attached to the mineral nanoparticle as defined above and described in the particular embodiments above. In another particular embodiment, the self-propulsion system comprises an enzyme attached to the metal layer as defined above and described in the particular embodiments above. In another particular embodiment, the self-propulsion system comprises an enzyme attached to the mineral layer as defined above and described in the particular embodiments above.

In a particular embodiment, the molecular drill and the self-propulsion system are attached to different points on the surface of the nanoparticle. In another particular embodiment, the molecular drill and the self-propulsion system are attached to the surface of the nanoparticle in diametrically opposite areas. In a preferred embodiment, the self-propulsion system comprises the molecular drill of the invention attached to its surface by means of a covalent bond.

Therapeutic Agent of the Invention

As will be understood by one skilled in the art, the therapeutic agent/agents of the invention is/are the therapeutic agent/agents comprised in the nanodevice of the invention.

In a particular embodiment, the at least one therapeutic agent comprises, preferably consists of, an antimicrobial compound and/or an antiseptic compound.

In a particular embodiment, the antimicrobial compound referred to in the present invention comprises, preferably consists of, an antibiotic and/or an antifungal.

The term "antibiotic", as it is used herein, refers to compounds that fight infections caused by bacteria in humans and animals by either killing the bacteria or hindering their growth and multiplication.

In a particular embodiment, the antibiotic referred to in the present invention is selected from the list consisting of vancomycin, cloxacillin, levofloxacin, gentamicin, rifampicin, clarithromycin, cefotaxime, imipenem, moxifloxacin, linezolid, ciprofloxacin, tobramycin, ceftazidime, colistin, piperacillin-tazobactam, imipenem, meropenem, amoxicillin, amoxicillin-clavulanic acid, metronidazole, clindamycin, azithromycin, dalbavancin, and combinations thereof.

Therefore, in a particular embodiment, the at least one therapeutic agent comprised on the surface of the nanoparticle of the invention comprises, preferably consists of, at least one antibiotic selected from the list of antibiotics indicated right above.

The term "antifungal", as it is used herein, refers to compounds with the ability to prevent the growth of certain fungi or cause their death.

In a particular embodiment, the antifungal referred to in the present invention is selected from the list consisting of fluconazole, voriconazole, micafungin, caspofungin, posaconazole, anidulafungin, clotrimazole, and combinations thereof.

Therefore, in a particular embodiment, the at least one therapeutic agent comprised on the surface of the nanoparticle of the invention comprises, preferably consists of, at least one antifungal selected from the list of antifungals indicated right above.

The term "antiseptic", as it is used herein, refers to compounds that inhibit the growth of or destroy microorganisms and can be applied to living tissues without harmful effects to the body, wherein microorganisms include bacteria, fungi, viruses, and protozoa.

In a particular embodiment, the antiseptic compound referred to in the present invention is selected from the list consisting of chlorhexidine, cetylpyridinium chloride, povidone iodine, sodium hypochlorite, and combinations thereof.

Therefore, in a particular embodiment, the at least one therapeutic agent comprised on the surface of the nanoparticle of the invention comprises, preferably consists of, at least one antiseptic selected from the list of antiseptics indicated right above.

In a particular embodiment, the at least one therapeutic agent comprised in the nanoparticle of the invention comprises, preferably consists of, combinations of different therapeutic agents. In a particular embodiment, the at least one therapeutic agent comprised in the nanoparticle of the invention comprises, preferably consists of, at least one antibiotic selected from the list of antibiotics indicated above, and at least one antifungal from the list indicated above. In another particular embodiment, the at least one therapeutic agent comprised in the nanoparticle of the invention comprises, preferably consists of, at least one antibiotic selected from the list of antibiotics indicated above, and at least one antiseptic from the list indicated above. In another particular embodiment, the at least one therapeutic agent comprised in the nanoparticle of the invention comprises, preferably consists of, at least one antifungal selected from the list of antifungals indicated above, and at least one antiseptic from the list indicated above. In another particular embodiment, the at least one therapeutic agent comprised in the nanoparticle of the invention comprises, preferably consists of, at least one antibiotic from the list indicated above, at least one antifungal from the list indicated above, and at least one antiseptic from the list indicated above.

In a particular embodiment, various therapeutic agents are located in a pore of the nanoparticle of the invention. In a particular embodiment, at least 1, 2, 3, 4, 5, 6, 7, 8, 9, 10, 15, 20, 50, 100 therapeutic agents, as defined in any of the particular embodiments above, are located in a pore of the nanoparticle of the invention.

In a particular embodiment, the amount of therapeutic agent is the corresponding therapeutically effective amount, wherein "therapeutically effective amount" refers to the amount of agent sufficient to provide the desired antiseptic, antibacterial, and/or antifungal effect and is generally determined, among others, by the characteristics of the component itself and the therapeutic effect to be achieved. It also depends on the subject to be treated, the severity of the infection to be treated, the format, route of administration, etc.

Although the needs of individuals vary, determining optimal ranges of therapeutically effective amounts for compounds in the context of the invention is within the common general experience of those skilled in the art. In general, the dose necessary to provide the desired therapeutic effect depends on age, health condition, sex, diet, weight, degree of infection, treatment frequency, nature and condition of the infection, nature and severity of the infection, medical condition of the subject, route of administration, pharmacological considerations such as activity, efficacy, pharmacokinetics, and toxicological profile of the agent in question and whether the agent is administered in combination with other medicinal products.

In a preferred embodiment, the amount of therapeutic agent comprised in a nanoparticle is between 1 and 100 $\mu$g/mg of nanoparticle; more preferably between 10 and 60 $\mu$g/mg of nanoparticle. Pharmaceutical Composition of the Invention

The second aspect of the invention relates to a pharmaceutical composition comprising the nanodevice according to the first aspect of the invention.

In a particular embodiment, the pharmaceutical composition comprises the nanodevice according to the first aspect of the invention, and a pharmaceutically acceptable adjuvant.

The term "pharmaceutically acceptable adjuvant" or "pharmaceutically acceptable excipient", as it is used herein, refers to a compound or combinations of compounds that is essentially non-toxic to a subject at the dose and concentration used, and is compatible with the other components of the medicinal product or pharmaceutical composition. Therefore, it is an inactive substance formulated together with the active ingredient (the nanodevice of the invention), of the medicinal product or pharmaceutical composition for the synthesis of compositions comprising said active ingredient. Synthesis of the compositions allows for proper and precise dispensing of the active ingredient when producing a dosage form. Excipients can also serve as enhancers, in other words, to facilitate the absorption of the active ingredient or its solubility, for example, or other pharmacokinetic consideration. Excipients can also be useful in the production process to facilitate the handling of the active substance in question such as, for example, to facilitate flowability and non-caking properties, to aid in *in vitro* stability, as well as to prevent denaturation throughout storage time. The selection of suitable excipients depends on the route of administration and the format of the medicinal product or pharmaceutical composition, as well as on the active ingredient, or other factors. An excipient can be a solid, semi-solid, liquid, diluent, encapsulation material, or an auxiliary formulation, that is non-toxic. Non-limiting examples of a pharmaceutically acceptable excipient include water, saline solution, alcohol, dextrose, vegetable oil, polyethylene glycol, gelatin, lactose, amylose, magnesium stearate, surfactants, silicic acid, viscous paraffin, talc, scented oil, fatty acid monoglycerides and diglycerides, fatty acid esters, hydroxymethyl cellulose, polyvinylpyrrolidone, or the like.

In a particular embodiment, particular definitions and embodiments of the first aspect of the invention apply to the second aspect of the invention. Medical Uses of the Invention

In a third aspect, the invention relates to the nanodevice according to the first aspect or pharmaceutical composition according to the second aspect of the invention, for use thereof as a medicinal product.

In a fourth aspect, the invention relates to the nanodevice according to the first aspect or pharmaceutical composition according to the second aspect of the invention, for use in the treatment of an infection in a subject, characterized in that the infected area in the subject comprises a biofilm produced by microorganisms or infectious agents that have invaded said infected area.

The term "medicinal product", as it is used herein, refers to a composition comprising a therapeutically effective amount of an agent, preferably the nanodevice of the first aspect of the invention. The medicinal product may further comprise a pharmaceutically acceptable excipient. The term "pharmaceutically acceptable excipient" has been defined in the second aspect of the invention and applies to the third aspect of the invention, wherein the active ingredient is the nanodevice of the first aspect of the invention.

The term "therapeutically effective amount" has been defined in the first aspect of the invention in relation to the therapeutic agent comprised in the nanoparticle of the invention and applies to the medicinal product of the third aspect of the invention, wherein the agent is the nanodevice of the first aspect. of the invention.

The term "infection", as it is used herein, refers to the penetration, development, or multiplication of an infectious agent in an organism. As a result, at least one area of an organism is invaded by an infectious agent. The term "infectious agent" refers to living organisms or biological entities which are capable of generating an infection, in other words, organisms or entities which, upon invading a tissue in an organism, cause a reaction to the presence thereof in said organism and can release toxins in said organism. Infectious agents can be bacteria, fungi, protozoa, viruses, and viroids. Infections can also be local (affecting a tissue or part of an organism) or systemic (affecting various tissues or parts of an organism). In a particular embodiment, the infectious agents referred to in the present invention are microorganisms that have invaded the infected area. The term "microorganism", as it is used herein, refers to organisms that can only be seen under a microscope and these organisms include bacteria, protozoa, algae, and fungi. Infections can occur in an isolated manner, or can give rise to complications that lead to more complex pathologies or diseases. They can also be part of the development or complication of a specific medical pathology or disease. For example, some infections can be the cause of gum diseases (periodontitis), inflammation of the vagina (vaginosis), nail diseases (onychomycosis), or halitosis (bad breath).

The term "subject" or "patient", as it is used herein, refers to an animal of any sex and age, and includes, but is not restricted to, domestic and farm animals, primates and humans. For example, it includes humans, non-human primates, cows, horses, pigs, sheep, goats, dogs, cats, or rodents. In a preferred embodiment, the subject is a female or male human of any age and ethnicity.

In a particular embodiment, the infection referred to in the present invention is characterized in that the infected area, I.e., the area invaded by infectious agents, comprises a biofilm generated by the infectious agents that have given rise to the infection. In a particular embodiment, the pH of the medium comprised inside the biofilm, i.e., between the biofilm and the wall of the infected tissue, is less than the pH of the medium outside the biofilm. In a particular embodiment, the pH of the medium comprised inside the biofilm is less than 7, 6.5, 6, 5.5, 5, 4.5, 4, 3.5, 3, 2.5, 2, 1.5, 1, preferably less than 5. In another particular embodiment, the pH of the medium comprised inside the biofilm is about 7, 6.5, 6, 5.5, 5, 4.5, 4, 3.5, 3, 2.5, 2, 1.5, 1, preferably about 5.

In a particular embodiment, the microorganisms that have invaded the infected area are at least one bacterium, a fungus, or a combination of both.

In a particular embodiment, microorganisms, bacteria, and/or fungi referred to in the present invention, preferably the microorganisms of the infection referred to in the present invention, form biofilms. The term "biofilm" has been defined in the first aspect of the invention and applies to the present inventive aspect. The formation of biofilms by microorganisms has been described in said definition of biofilm, as well as methods to detect the presence of a biofilm, and the microorganisms generating same.

In a particular embodiment, the at least one bacterium referred to in the present invention is of a genus which is selected from the list consisting of *Staphylococcus* spp., *Pseudomonas* spp., and combinations thereof.

In another particular embodiment, bacteria of the genus *Staphylococcus spp.* referred to in the present invention are of a species which is selected from the list consisting of *Staphylococcus aureus (S. aureus), Staphylococcus epidermidis (S. epidermidis), Staphylococcus lugdunensis (S. lugdunensis), Staphylococcus saprophyticus (S. saprophyticus), Staphylococcus hominis (S. hominis), Staphylococcus haemolyticus (S. haemolyticus), Staphylococcus capitis (S. capitis), Staphylococcus capitis (S. capitis capitis), Staphylococcus warneri (S. warnen),* and combinations thereof. In a preferred embodiment, the bacterium referred to in the present invention is of the species *S. aureus.*

In another particular embodiment, bacteria of the genus *Pseudomonas spp.* referred to in the present invention are of a species which is selected from the list consisting of *Pseudomonas aeruginosa (P. aeruginosa), Pseudomonas pseudomallei (P. pseudomallei), Pseudomona mirabilis (P. mirabilis), Pseudomonas oryzihabitans (P. oryzihabitans), Pseudomonas fluorecens (P. fluorecens), Pseudomonas putida (P. putida), Pseudomonas stutzeri (P. stutzeri), Pseudomonas pickettii (P. pickettii),* and combinations thereof. In a preferred embodiment, the bacterium referred to in the present invention is of the species *P. aeruginosa.*

In another particular embodiment, the at least one fungus referred to in the present invention is of a genus which is selected from the list consisting of *Candida* spp., *Aspergillus spp., Cryptococcus spp.,* and combinations thereof.

In a particular embodiment, the fungi of the genus *Candida* spp. are selected from the list consisting of *Candida albicans (C. albicans), Candida Auris (C. Auris), Candida glabrata (C. galabrata), Candida parapsilosis (C. parapsilosis), Candida tropicalis (C. tropicalis),* and combinations thereof. In a preferred embodiment, the fungus referred to in the present invention is of the species *Candida albicans (C. albicans).*

In another particular embodiment, the fungi of the genus *Aspergillus spp.* are selected from the list consisting of *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus terreus, Aspergillus tubingensis, Aspergillus ustus, Aspergillus versicolor,* and combinations thereof.

In another particular embodiment, the fungi of the genus *Cryptococcus spp.* are selected from the list consisting of *Cryptococcus diffluens, Cryptococcus dimennae, Cryptococcus flavus, Cryptococcus gastricus, Cryptococcus gattii, Cryptococcus laurentii, Cryptococ-*

*cus luteolus, Cryptococcus macerans, Cryptococcus magnus, Cryptococcus neoformans, Cryptococcus oeirensis, Cryptococcus podzolicus, Cryptococcus skinneri, Cryptococcus terreus, Cryptococcus victoriae,* and combinations thereof.

In a particular embodiment of the medical use of the invention, the at least one therapeutic agent comprised in the nanodevice of the invention comprises, preferably consists of, at least one antibiotic selected from the list of antibiotics indicated in inventive aspect 1. In another particular embodiment of the medical use of the invention, the at least one therapeutic agent comprised in the nanodevice of the invention comprises, preferably consists of, at least one antifungal selected from the list of antifungals indicated in inventive aspect 1. In another particular embodiment of the medical use of the invention, the at least one therapeutic agent comprised in the nanodevice of the invention comprises, preferably consists of, at least one antiseptic selected from the list of antiseptics indicated in inventive aspect 1. In another particular embodiment, the at least one therapeutic agent comprised in the nanodevice of the invention comprises, preferably consists of, at least one antibiotic selected from the list of antibiotics indicated in inventive aspect 1, and at least one antifungal selected from the list of antifungals indicated in inventive aspect 1. In another particular embodiment, the at least one therapeutic agent comprised in the nanodevice of the invention comprises, preferably consists of, at least one antibiotic selected from the list of antibiotics indicated in inventive aspect 1, and at least one antiseptic selected from the list of antiseptics indicated in inventive aspect 1. In another particular embodiment, the at least one therapeutic agent comprised in the nanodevice of the invention comprises, preferably consists of, at least one antifungal selected from the list of antifungals indicated in inventive aspect 1, and at least one antiseptic selected from the list of antiseptics indicated in inventive aspect 1. In another particular embodiment, the at least one therapeutic agent comprised in the nanodevice of the invention comprises, preferably consists of, at least one antifungal selected from the list of antifungals indicated in inventive aspect 1, at least one antiseptic selected from the list of antiseptics indicated in inventive aspect 1, and at least one antibiotic selected from the list of antibiotics indicated in inventive aspect 1.

As will be understood by one skilled in the art, the therapeutic agent to be used in the treatment of an infection depends on the infectious agents or microorganisms causing the infection.

In a particular embodiment of the medical use of the invention, the microorganisms comprise a bacterium, preferably of the genus *Staphylococcus* spp. and/or *Pseudomonas* spp., and the at least one therapeutic agent comprised in the nanodevice of the invention comprises, preferably consists of, an antibiotic selected from the list consisting of vancomycin, cloxacillin, levofloxacin, gentamicin, rifampicin, clarithromycin, cefotaxime, imipenem, moxifloxacin, linezolid, dalbavancin, ciprofloxacin, tobramycin, ceftazidime, colistin, ciperacillin-tazobactam, imipenem, meropenem, amoxicillin, amoxicillin-clavulanic acid, metronidazole, clindamycin, azithromycin, and combinations thereof.

In a particular embodiment of the medical use of the invention, the microorganisms comprise a bacterium of the genus *Staphylococcus* spp. and the at least one therapeutic agent comprised in the nanodevice of the invention comprises, preferably consists of, an antibiotic selected from the list consisting of vancomycin, cloxacillin, levofloxacin, gentamicin, rifampicin, clarithromycin, cefotaxime, imipenem, moxifloxacin, linezolid, dalbavancin, and combinations thereof.

In another particular embodiment of the medical use of the invention, the microorganisms comprise a bacterium of the species S. *aureus* and the at least one therapeutic agent comprised in the nanodevice of the invention comprises the antibiotic vancomycin, preferably it is the antibiotic vancomycin.

In a particular embodiment of the medical use of the invention, the microorganisms comprise a bacterium of the genus *Pseudomonas* spp. and the at least one therapeutic agent comprised in the nanodevice of the invention comprises, preferably consists of, an antibiotic selected from the list consisting of ciprofloxacin, tobramycin, ceftazidime, colistin, ciperacillin-tazobactam, imipenem, meropenem, amoxicillin, amoxicillin-clavulanic acid, metronidazole, clindamycin, azithromycin, and combinations thereof.

In a particular embodiment of the medical use of the invention, the microorganisms comprise fungi, preferably of the genus *Candida* spp., *Aspergillus spp.,* and/or *Cryptococcus,* and the at least therapeutic agent comprised in the nanodevice comprises, preferably consists of, an antifungal selected from the list consisting of fluconazole, voriconazole, micafungin, caspofungin, posaconazole, anidulafungin, clotrimazole, and combinations thereof.

In a particular embodiment of the medical use of the invention, the microorganisms comprise a fungus of the genus *Candida* spp., and the least one therapeutic agent comprised in the nanodevice comprises, preferably consists of, an antifungal selected from the list consisting of fluconazole, voriconazole, micafungin, caspofungin, posaconazole, anidulafungin, clotrimazole, and combinations thereof. Preferably, the antifungal used is micafungin.

In another particular embodiment of the medical use of the invention, the microorganisms comprise a fungus of the genus *Aspergillus spp.* and the least one therapeutic agent comprised in the nanodevice comprises, preferably consists of, an antifungal which is selected from the list consisting of voriconazole and/or posaconazole.

In another particular embodiment of the medical use of the invention, the microorganisms comprise a fungus of the genus *Cryptococcus* spp. and the least one therapeutic agent comprised in the nanodevice comprises, preferably consists of, the antifungal fluconazole.

In a particular embodiment of the medical use of the invention, the infection is a local infection in a region or tissue in contact with the external environment or close to the outside environment. For example, it can be located in any of the layers of the skin (epidermis, dermis, or the fat that is part of the skin) of a subject, of the mucosa (squamous epithelium or lamina propria), or even the submucosa. It can also be located in lower layers but exposed to the outside environment due to an incision or wound that affects said lower layers. In addition, infections in the nails, as well as in internal regions, such as abscesses or mastitis, could also be treated. In a particular embodiment, the infection is an endodontic infection, supragingival infection, subgingival infection, infection in a tissue surrounding a dental implant, a prosthesis, preferably in contact with a layer of the skin or the mucosa, preferably buccal mucosa, a catheter or medical device, preferably in contact with a layer of the skin or the mucosa, preferably buccal mucosa, an infection of the nail structure, including the nail, the cuticle, the perinichium or hyponychium periungual edge, eponychium, nail bed, or root of the nail, or an infection of the vaginal mucosa. In a particular embodiment, the infection is part or consists of a pathology, wherein said pathology results from, consists of, or has worsened due to, an infection caused by biofilms. In a preferred embodiment, the pathology is selected from the list consisting of endodontic infection, caries, gingivitis, periodontitis, halitosis, peri-implantitis, vaginosis, onychomycosis, and combinations thereof.

In a particular embodiment, the mucosas referred to in the present invention comprise mucosas selected from the list consisting of buccal mucosa, throat mucosa, stomach mucosa, intestinal mucosa, nasal mucosa, respiratory tract mucosa, vaginal mucosa, and combinations thereof.

The term "pathology", as it is used herein, refers to an anatomical and/or physiological alteration or disorder of a tissue and/or organ of a subject, as well as the symptoms and signs through which said disorders are presented and the causes causing same.

The term "endodontic infection", as it is used herein, refers to an infection which affects the root canal system. It is the main etiological agent of apical periodontitis.

The term "caries", as it is used herein, refers to permanently damaged areas on the surface of the teeth that develop into small openings or holes. They are generally caused by the action of certain bacteria in the buccal cavity.

The term "periodontitis", as it is used herein, refers to an infection of the gums which damages the soft tissue and may destroy the bone supporting the teeth without treatment. Periodontitis can cause the loosening or the loss of teeth.

The term "halitosis", as it is used herein, refers to bad breath resulting from the accumulation of bacteria in the mouth or between the teeth, or food debris around the gums and tongue, which in turn trigger the bloom of caries-causing bacteria in the mouth.

The term "vaginosis", as it is used herein, refers to a type of vaginal inflammation caused by the excessive growth of microorganisms, generally bacteria or fungi, found naturally in the vagina, which upsets the natural balance.

The term "onychomycosis", as it is used herein, refers to an alteration in the nails caused by the invasion of pathogenic or saprophytic fungi in the nail structure of the hands and/or feet. It is the main cause of nail disease in developed countries, in which it accounts for about 30-50% of nail disorders.

For the operation of the self-propulsion system of the nanodevice of the invention, it is necessary to provide a fuel, as defined in the first aspect of the invention, for most self-propulsion systems.

In a particular embodiment of the medical use of the invention, the self-propulsion system of the nanodevice comprises a metal selected from the list consisting of Pt, Ag, Ir, Ni, Mg, and combinations thereof, and/or the mineral $MnO_2$, and the fuel is hydrogen peroxide.

In another particular embodiment of the medical use of the invention, the self-propulsion system of the nanodevice comprises the enzyme urease and the fuel is urea.

In another particular embodiment of the medical use of the invention, the self-propulsion system of the nanodevice comprises the enzyme glucose oxidase and the fuel is glucose.

In another particular embodiment, the self-propulsion system of the nanodevice comprises the enzyme glucose oxidase and no fuel is administered, in particular, no glucose is administered.

In another particular embodiment, the self-propulsion system of the nanodevice comprises Zn and no fuel is administered.

Therefore, in a particular embodiment of the medical use of the invention, the nanodevice of the invention or the pharmaceutical composition of the invention and a fuel are administered in the infected area of the subject, wherein the self-propulsion system and the fuel is a combination indicated in any of the particular embodiments indicated right above. In another particular embodiment, the nanodevice of the invention or the pharmaceutical composition of the invention is administered at the same time as a fuel, wherein the self-propulsion system and the fuel is a combination indicated in any of the particular embodiments indicated right above. In another particular embodiment, the nanodevice of the invention or the pharmaceutical composition of the invention is administered in the infected area of the subject, and then the fuel is administered in said area, wherein the self-propulsion system and the fuel is a combination indicated in any of the particular embodiments indicated right above. In a particular embodiment, the fuel is administered in the infected area for 5 s, 10 s, 30 s, 1 min, 2 min, 3 min, 5 min, 10 min, 15 min, 30 min, 45 min, 60 min, 90 min, 120 min, 3 h, 4 h, 5 h, 6 h, 7 h, 8 h, 12 h, 24 h, 36 h, 48 h, 72 h, after administering the nanodevice of the invention or the pharmaceutical composition in the infected area of the subject, wherein the self-propulsion system and the fuel is a combination indicated in any of the particular embodiments indicated right above.

In a particular embodiment of the medical use of the invention, the nanodevice of the invention or the pharmaceutical composition of the invention is administered in the infected area of the subject, wherein the self-propulsion system comprises the enzyme glucose oxidase and/or Zn, and no fuel is administered in the infected area. In a particular embodiment, in a particular embodiment of the medical use of the invention, the nanodevice of the invention or the pharmaceutical composition of the invention is administered in the infected area of the subject, wherein the self-propulsion system comprises the enzyme glucose oxidase and no fuel is administered in the infected area, preferably no glucose fuel is administered. In another particular embodiment, in a particular embodiment of the medical use of the invention, the nanodevice of the invention or the pharmaceutical composition of the invention is administered in the infected area of the subject, wherein the self-propulsion system comprises Zn and no fuel is administered in the infected area.

In a preferred embodiment of the medical use of the invention, the amount of nanodevice administered is 0.1-10 mg/ml, 0.2-7 mg/ml, 0.25-5 mg/ml, 0.3-4 mg/ml, 0.4-3 mg/ml, 0.5-2 mg/ml, 0.5-1.5 mg/ml, preferably 0.5-1.5 mg/ml, with respect to the total volume of a solution in which it is administered. In a preferred embodiment, the solution in which it is administered comprises a pharmaceutically acceptable adjuvant as defined in inventive aspect 2. In another preferred embodiment, the solution in which it is administered comprises water, preferably distilled water. In an even more preferred embodiment, the solution in which the nanodevice of the invention is administered comprises sterile distilled water. Preferably, the amount of nanodevice of the invention considered necessary, depending on the concentration of therapeutic agent that it has, as well as on the type of biofilms to be treated, can be applied repeatedly.

In another preferred embodiment of the medical use of the invention, the amount of fuel administered is between 0.2-10 $\mu$l/ml, 0.3-7 $\mu$l/ml, 0.5-5 $\mu$l /ml, 0.5-4 $\mu$l /ml, 0.5-3 $\mu$l /ml, 0.5-2.5 $\mu$l /ml, preferably 0.5-2.5 $\mu$l/ml, with respect to the volume of a solution in which it is administered, wherein the administered fuel comprises any of those indicated in the particular embodiments indicated above. In a preferred embodiment, the solution in which the fuel is administered comprises any of those indicated in the definition of pharmaceutically acceptable adjuvant in the second aspect of the invention, preferably distilled water. In an even more preferred embodiment, the solution containing the fuel to be administered comprises sterile distilled water.

In a particular embodiment, the third aspect of the invention comprises the kit of the invention for use thereof in medicine. In a preferred embodiment, particular embodiments of the third aspect of the invention apply to the kit of the invention for use thereof in medicine.

In another particular embodiment, the fourth aspect of the invention comprises the kit of the invention for use thereof in the treatment of an infection in a subject, characterized in that the infected area in the subject comprises a biofilm produced by microorganisms or infectious agents that have invaded said infected area. In a preferred embodiment, the particular embodiments of the fourth aspect of the invention apply to said medical use of the kit of the invention.

The particular embodiments and definitions of the first and second aspects of the invention apply to the third and fourth aspects of the invention. <u>Use of the Nanodevice or of the Pharmaceutical Composition</u>

In a fifth aspect, the invention relates to the use of the nanodevice according to the first aspect of the invention, or of the pharmaceutical composition according to the second aspect of the invention to reduce the number of living microorganisms in a sample *in vitro* or on the surface of an inert material *ex vivo*, wherein said microorganisms have formed a biofilm.

The term "biofilm", and the microorganisms that can form biofilms, have been defined in the preceding inventive aspects and apply to the present inventive aspect. In a particular embodiment, the microorganisms referred to in the fifth aspect of the invention are those defined and described in the particular embodiments of any of the preceding aspects of the invention.

The term "reduce the number of living microorganisms", as it is used herein, refers to the number of microorganisms capable of multiplying, or of forming a suitable environment for the multiplication of microorganisms, particularly for the multiplication of microorganisms of the species thereof, in a given space being reduced by at least 10%, 20%, 30%. 40%, 50%, 60%, 70%, 80%, 90%, 100%. Methods for determining variation in the amount of living microorganisms in a given space are commonly known to one skilled in the art. Non-limiting examples of said methods include determining the amount of microorganisms present in a sample taken from a surface where the microorganisms are or may be located, before and after the expected variation occurs (i.e., before and after applying the nanodevice of the invention or the pharmaceutical composition of the invention). After taking each of the samples, the amount of microorganisms present in the sample is determined by methods commonly known to one skilled in the art for determining the amount of microorganisms in a given sample. Said methods include, in a non-limiting manner, transferring the sample in question to a medium suitable for the growth of microorganisms, incubating the sample in said medium for a certain time and at a certain temperature, and performing determination by turbidimetry, plate dilution, plate dripping by sealing, or counting the amount of microorganisms present in the sample in a Neubauer counting chamber. The difference between the amount of microorganisms in the sample taken before and after the occurrence of the expected variation allows determining variation in the number of microorganisms in question in the area from which the samples have been obtained. Culture media, times, and temperatures for certain microorganisms are commonly known to one skilled in the art. Non-limiting examples of said methods include lysogeny broth (abbreviated as LB) or eosin methylene blue Agar (abbreviated as EMB), particularly useful for the growth of bacteria, o Vogel-Johnson agar, mannitol salt agar, BHI agar, Sabouraud agar, or Baird-Parker agar, particularly useful for the growth of bacteria, preferably *S. aureus.*

In a particular embodiment, the reduction in the number of living microorganisms in a sample *in vitro* or on the surface of an inert material *ex vivo* comprises reducing by at least 10%, 20%, 30%. 40%, 50%, 60%, 70%, 80%, 90%, 100 %, preferably at least 90%, the number of said microorganisms in said sample *in vitro* or on the surface of an inert material. Methods for determining said reduction have been provided in the definition of the expression "reduce the number of living microorganisms" above.

As will be understood by one skilled in the art, by reducing the amount of living microorganisms in a sample *in vitro* or on the surface of an inert material *ex vivo,* as described, said sample or surface is disinfected.

The term "sample *in vitro",* as it is used herein, refers to a sample of any type, such as a sample already extracted from a subject, a medium of interest, a culture medium of interest, and stored in an insulated and sterile container.

The term "inert material *ex vivo"* refers to any material comprised on the outside of a subject's body. Said material can be part of a prosthesis, a dental prosthesis, an implant, a dental implant, a catheter, a pacemaker, a laboratory utensil, a medical utensil, a work surface, a work material, a surface in food facilities, kitchens, cookware, etc.

In a particular embodiment, the fifth aspect of the invention comprises the use of the kit of the invention to reduce the number of living microorganisms in a sample *in vitro* or on the surface of an inert material *ex vivo,* wherein said microorganisms have formed a biofilm. In a preferred embodiment, the particular embodiments of the fifth aspect of the invention apply to said use of the kit of the invention.

The particular definitions and embodiments of the first, second, third, and fourth aspects of the invention apply to the fifth aspect of the invention. Kit of the Invention In a sixth aspect, the invention relates to a kit comprising the nanoparticle of the invention, the molecular drill of the invention, the therapeutic agent of the invention, and the self-propulsion system of the invention.

The term "kit", as it is used herein, refers to a product that contains different components necessary to obtain the nanodevice of the invention, packaged to allow transport and storage. Furthermore, the kits of the invention may contain instructions for using the different components found in the kit simultaneously, sequentially, or separately. Said instructions can be in the form of printed material or in the form of an electronic support to store instructions that can be read or understood, such as, for example, electronic storage media (magnetic disks, pendrives or chips), or optical media (for example, CD-ROM, DVD), or an audio material. Furthermore, or alternatively, the media may contain Internet addresses that provide said instructions.

In another particular embodiment, the kit of the invention comprises at least one fuel as defined in the present invention. In a preferred embodiment, it comprises the at least one suitable fuel for the self-propulsion system comprised in the kit.

In another particular embodiment, the kit comprises the combinations of self-propulsion system and fuel indicated in the particular embodiments of the medical use of the invention. Therefore, particular embodiments of the third aspect of the invention specifying specific combinations of fuel and self-propulsion systems apply to the sixth aspect of the invention, replacing the expression "medical use of the invention" with "kit of the invention" and "self-propulsion system of the nanodevice" with "self-propulsion system of the invention".

In another particular embodiment, the kit of the invention further comprises a pharmaceutically acceptable adjuvant, as defined in the second aspect of the invention.

In a particular embodiment, the components comprised in the kit of the invention described in this section 5 of the invention each form at least 10%, 20%, 30%, 40%, 50%, 60%, 70%, 80%, 90%, or 100% of the total components of the kit of the invention.

The definition and particular embodiments of the first, second, third, fourth, and fifth aspects of the invention apply to the sixth aspect of the invention.

In a particular embodiment, the term "comprises" is replaced with "consists of" in any aspect of the invention. In another particular embodiment, the term "consists of" is replaced with "comprises" in any aspect of the invention.

The following examples and drawings are illustrative and are in no case intended to limit the invention.

**Methods and materials**

**Reagents**

n-cetyltrimethylammonium bromide (CTABr), tetraethyl orthosilicate (TEOS), dihydrogen hexachloroplatinate (IV) ($H_2PtCl_6$), polyvinylpyrrolidone (PVP), ascorbic acid, paraffin, (3-mercaptopropyl)trimethoxysilane, (3-oodopropyl)trimethoxysilane, benzimidazole (BENZ), triethylamine (TEA), vancomycin ($C_{66}H_{75}Cl_2N_9O2_4$), ficin, N-(3-dimethylaminopropyl)-N'-ethylcarbodiimide hydrochloride (EDC), N-hydroxysuccinimide (NHS), □-ciclodextrin (□CD), rhodamine B (Rh), 2,2'-azino-bis(3-ethylbenzothiazoline-6-sulfonic acid (ABTS), trifluoroacetic acid (TFA), and bovine milk casein were obtained from Sigma Aldrich. Alexa Fluor 680-dextran conjugate, bicinchoninic acid (BCA) assay kit, hydrogen peroxide (30%), and SYTO9 were obtained from Thermo Fisher. Sodium hydrogen phosphate monohydrate, disodium hydrogen phosphate heptahydrate, sodium acetate, sodium hydroxide (NaOH), ethanol, toluene, chloroform, dimethyl sulfoxide (DMSO), acetonitrile, phenol, sulfuric acid, and acetic acid were obtained from Scharlau. Trypticasein soy agar (TSA) plates, Liquid trypticasein soy medium (TSB).

## General methods and instruments

Powder X-ray diffraction (PXRD) analyses were carried out using a D8 Advance Seifert 3000TT diffractometer using CuK$\alpha$ radiation at low angles ($1.5 < 2\theta > 7$, with steps of 0.04 degrees and 3s for each step), and high angles ($35 < 2\theta > 80$ with steps of 0.04 degrees and 1s for each step). The $N_2$ adsorption-desorption isotherms were obtained using a Micromeritics TriStar II Plus automated analyzer. The nanomotors were vacuum degassed at 90 or 120°C overnight. The specific surface area was calculated based on the adsorption data in the low-pressure range using the Brunauer-Emmett-Teller (BET) model, whereas the pore size was determined following the Barrett-Joyner-Halenda (BJH) method. Thermogravimetric (TGA) analyses were performed with the TA Instruments SDTQ600 apparatus in an oxidizing atmosphere (air, 80 ml min$^{-1}$) and a heating program between 393 and 1273°C at 10°C min$^{-1}$ followed by a 30-minute isothermal heating leap to 1273°C. Fourier transform infrared (FTIR) spectroscopy measurements were carried out with Tensor 27 (Bruker). Dynamic light scattering (DLS) and zeta potential measurements were performed using ZetaSizer Nano ZS (Malvern). Nanoparticle tracking experiments were performed with Nanosight NS300 (Malvern). UV-visible measurements were carried out on a JASCO V-650 spectrophotometer. Fluorescence measurements were performed with a JASCO FP-8500 spectrophotometer. Element mapping was carried out by means of scanning electron microscopy coupled with energy dispersive x-ray electron spectroscopy (STEM-EDX) using a JEM 2100F instrument to that end. Transmission electron microscopy images of the nanomotors were obtained using a JEOL TEM-2100F electron microscope. Optical dispersion (OD) measurements were performed with a Tecan spectrophotometer (Durham, NC, USA). Bacterial cell index experiments were performed with an xCELLigence real-time analyzer (Agilent). Confocal microscopy images were obtained with a Leica TCS SP8 AOBS inverted laser confocal microscope. Confocal microscopy images of S. *aureus* biofilms were taken with Hitachi S-4800 (Electron Microscopy Service of the University of Valencia, Spain).

## Synthesis of Nanoparticles

### Mesoporous Silica Nanoparticles (MSNs)

1 g of CTABr is dissolved in 480 ml of deionized water. Next, 3.5 ml of a 2 M NaOH solution is added to the mixture and the temperature is set at 80°C. Once this temperature is reached, 5 ml of TEOS are added dropwise to the solution, which is left under stirring for 2 h, obtaining a white precipitate as a result. Then, the solid obtained is isolated by means of centrifugation, washed with deionized water, and left to dry at 70°C. Finally, the solid is calcined for 5 h at 550°C under an oxidizing atmosphere to obtain the final nanoparticles, **MSNs.**

### Platinum Nanodendrites (PtNDs)

164 mg of $H_2PtCl_6$ and 20 mg of PVP are mixed in 20 ml of deionized water. At the same time, 350 mg of ascorbic acid are dissolved in 10 ml of distilled water. This mixture is poured dropwise on the $H_2PtCl_6$ solution. After this, the temperature is increased to 45°C and the reaction is left under magnetic stirring for 3 h. The color of the solution changes from pale yellow to black revealing the successful synthesis of platinum nanodendrites, **PtNDs.**[47]

### Janus Pt-MSN Nanomotors (NMs)

The synthesis of Janus Pt-MSN nanomotors was carried out following the method previously described by the present inventors.[27,28] 180 mg of MSN are dispersed in a 6.7% aqueous ethanol solution. 208 $\mu$l of a CTAB solution (1 $\mu$M) are added and the temperature is set at 75°C. Then, 1 g of paraffin is added. Once it has melted, an Ultra-Turrax T-8 (IKA) is used for 10 min to homogenize the sample. Next, the reaction is left under magnetic stirring at 75°C for 1 h so that a Pickering emulsion is created. After this time, the mixture is removed from heat and left to cool, adding at this time 200 $\mu$l of (3-mercaptopropyl)trimethoxysilane and 10 ml of ethanol. The reaction mixture is left under magnetic stirring for 3 h. Then, the solid obtained is isolated by centrifugation and washed twice with methanol. Lastly, the mercaptosilane-functionalized face of the MSNs reacts with previously synthesized PtNDs, under magnetic stirring at room temperature overnight, to obtain Janus Pt-MSN nanomotors, **NMs,** which will be filtered, washed with chloroform, and dried.

### Functionalization of the surface of NMs/MSNs with benzimidazole groups (NM$_{BENZ}$/MSN$_{BENZ}$)

The mesoporous face of the MSNs and NMs were functionalized with benzimidazole groups for subsequent reaction with ficin-linked β-cyclodextrins to form inclusion complexes that act as pH-activated nanovalves. To that end, 60 mg of MSMs or NMs are suspended in 4 ml of anhydrous ACN and reacted with 60 $\mu$l of (3-iodopropyl)trimethoxysilane for 5.5 h. The solids are isolated by means of centrifugation and washed with toluene. To prepare a saturated benzimidazole solution, 0.25 g is mixed with 330 $\mu$l of TEA and 20 ml of toluene and heated at 80°C until complete dissolution. Then, 10 ml of this benzimidazole solution is added to the MSNs or NMs and the mixture is refluxed at 80°C under magnetic stirring for 3 days. Lastly, the solids obtained $NM_{BENZ}$ and $MSN_{BENZ}$ are centrifuged, washed with toluene, and dried at 37°C.

## Synthesis of Ficin Modified with β-cyclodextrin (F-βCD)

25 mg of ficin are dissolved in 4 ml of 100 mM phosphate buffer pH 6 at 4°C and mixed with 21 mg of EDC and 21 mg of NHS for 30 min to activate the acid groups of the protein. Then, 13 mg of $\beta CD-NH_2$ (synthesized as described above)[48] are added to the mixture which is stirred magnetically for 12 h to generate amide bonds between the amino groups of $\beta CD-NH_2$ and the activated acid groups of the protease ficin. Lastly, the ficin-β-cyclodextrin conjugate (F-βCD) obtained is dialyzed with cold 100 mM phosphate buffer, pH 7.5, using Amicon Ultra-05 centrifuge filters (3 kDa).[49] F-□CD is stored at 4°C until use.

## Ficin-Capped Janus Pt-MSN Nanomotors Loaded with Vancomycin ($NM_{VF}$)

The NM pores were loaded with the antibiotic vancomycin. In a typical synthesis, 50 mg of $NM_{BENZ}$ are mixed with 7.5 mg of vancomycin in 4 ml of 100 mM PBS pH 7.5 and magnetically stirred overnight. Then, the solid is isolated by centrifugation and resuspended again in PBS. To link the molecular gate (F-□CD), $NM_{BENZ}$ loaded with vancomycin were reacted with 12.5 mg of F-βCD at 4°C overnight. Lastly, $NM_{VF}$ was centrifuged and washed several times with 100 mM PBS pH 7.5.

## Cyclodextrin-Capped Control Janus Pt-MSN Nanomotors Loaded with Vancomycin ($NM_V$)

To synthesize the ficin-free control nanomotor, $NM_V$, 15 mg of $NM_{BENZ}$ loaded with vancomycin are reacted with 5 mg of β-CD in 3 ml of 100 mM PBS pH 7.5 overnight.

## Ficin-Capped Control Janus Pt-MSN Nanomotors ($NM_F$)

To synthesize the vancomycin-free control nanomotor, $NM_F$, 15 mg of $NM_{BENZ}$ are reacted with 3.75 mg of F-βCD in 3 ml of 100 mM PBS pH 7.5 at 4°C overnight.

## Ficin-Capped Control MSNs loaded with Vancomycin ($NM_{MSN}$)

The PtND-free control nanodevice, $NM_{MSN}$, is prepared following the same method followed for $NM_{VF}$, using $MSN_{BENZ}$ instead of $NM_{BENZ}$.

## Synthesis of the Vancomycin-Rhodamine B Conjugate (V-Rh)

For UV-visible and CLM viewing purposes, vancomycin was modified with the dye rhodamine B (V-Rh). 5 mg of rhodamine B (10 □mol, 1.4 eq.) were activated with an excess of EDC (15 mg, 115 □mol) y NHS (22 mg, 190 $\mu$mol) in 2 ml of 100 mM PBS pH 6 for 30 min. Next, 10 mg of vancomycin (7 □mol, 1.0 eq.) were added to the mixture and left to react at 4°C overnight.

## Ficin-capped Janus Pt-MSN Nanomotors Loaded with Vancomycin-Rhodamine ($NM_V.RhF$)

The solid $NM_{V-RhF}$ was prepared following the same method used for the synthesis of $NM_{VF}$. However, the pores have been loaded with V-Rh. All nanodevices were stored at 4°C until use. SI-Table 1 summarizes all the nanodevices used in the study.

## Optical Video Acquisition and Movement Analysis by means of MSD analysis

To evaluate the movement capacity of the nanomotors, nanoparticle individual tracking analysis (NTA) was applied. $NM_{VF}$ are dispersed in 100 mM PBS at pH 7.5 at a concentration of 0.002 mg $ml^{-1}$, subjected to ultrasonication, and introduced by means of a 1 ml syringe into the chamber of the Nanosight NS300 device (total volume 800 $\mu$l) at a temperature of 25°C, in the presence of different concentrations of the fuel, $H_2O_2$ (0%, 0.035%, 0.105%, and 0.2%). The fuel solutions were already present in the chamber when the nanodevices were added to avoid any drift interference. Next, 5 videos of 30 s at a speed of 30 frames $s^{-1}$ are recorded with a sCMOS camera coupled to the optical microscope. The NTA 3.0 software was used to analyze the x and y coordinates of 15 nanomotors (between 100 and 400 nm in size) over time ($\Delta t = 0.4$ s), to calculate by applying **equation 1** the mean squared displacement (MSD) of each of them. The $D_{eff}$ of nanomotors in the presence of $H_2O_2$ and $D_0$ for nanomotors in the absence of $H_2O_2$ were obtained by representing MSD *vs* $\Delta t$, through the linear component of the Stokes-Einstein equation, **equation 2,** using an R code of own development.

Furthermore, the movement of **$NM_{V-RhF}$** in the presence of S. *aureus* cells was checked. 1 mg $ml^{-1}$ of $NM_{V-RhF}$ and 0.15% of $H_2O_2$ were incubated with a preformed biofilm of *S. aureus* with 6 h maturity in an IBIDI μ-Slide I Luer microfluidics device, and recording was performed at 10 min with CLSM (SI-video 1, green: rhodamine of **NM$_{V-RhF}$**, red: polysaccharide matrix labeled with the Alexa Fluor 680-dextran conjugate).

## Equation 1:

$$MSD \equiv \langle (x_t - x_0)^2 \rangle = \frac{1}{N} \sum_{i=0}^{N} \left( x^i(t) - x^t(0) \right)^2$$

(wherein N is the average number of nanoparticles, $x^i$ and $x^t$ are position vectors of the nanoparticles at different times).

## Equation 2:

$$MSD = (4D_0)\, \Delta t \,/\, MSD = (4D_{eff})\, \Delta t$$

(wherein $D_0$ is the diffusion coefficient of the nanomotors without fuel; and $D_{eff}$ is the effective diffusion coefficient of the nanomotors in the presence of fuel).

## Equation 3:

$$D = \frac{T K_B}{6\pi\eta R_h}$$

(wherein $\eta$ is viscosity, $10^{-3}$ Pa s, $k_B$ is the Boltzmann constant, T is temperature, 25°C, and $R_h$ the hydrodynamic radius, 185.65 nm)

### Load Control Release Assays

To study the ability of nanomotors to transport vancomycin, 1 mg of **NM$_{V-RhF}$** is suspended in 1 ml of 100 mM PBS pH 7.5 (blank) and 1 mg of NM$_{V-RhF}$ is suspended in 1 ml of 100 mM PBS pH 4.5. The mixtures are stirred in a shaker at 37°C. At different times (0, 30, 60, 90, and 120 min), samples are centrifuged (5 min, 12,500 rpm, 4°C) to remove the **NM$_{V-RhF}$**. Lastly, the emission fluorescence of the V-Rh conjugate released into the supernatant is measured ($\lambda_{exc}$ = 546 nm, $\lambda_{em}$ = 568 nm). Furthermore, the release properties of NM$_V$ were also evaluated. The method followed was the same as that explained above. However, the supernatant was dialyzed (2 centrifugation cycles of 10 min using Amicon Ultra-05 centrifuge filters, 3 kDa) to remove molecular gate (βCD). Furthermore, released vancomycin was detected by means of UV-vis spectroscopy ($\lambda$ = 264 nm) at 0 and 24 h. The experiments, depicted in Supplementary Figure 9, were performed in triplicate.

### Strains and Bacterial Growth Conditions

The experiments were performed using *Sta-*

*phylococcus aureus* strain Sa240 (*S. aureus spp. aureus* Rosenbach 1884). The strain was seeded in TSA and TSB media and cultured overnight at 37°C with vigorous stirring at 120 rpm.

### Effect of H$_2$O$_2$ on Planktonic and Biofilm Growth

To evaluate the effect of H$_2$O$_2$ on the planktonic growth of Sa240, 100 μl (OD$_{600}$ = 0.175) of bacterial suspensions were added to 96-well plates in triplicate for each condition. Subsequently, 100 μl of H$_2$O$_2$ diluted in TSB supplemented with 0.25% filter-sterilized D-glucose (TSB-glu) were added, reaching final concentrations of H$_2$O$_2$ of 0.35%, 0.30%, 0.25%, 0.20%, 0.15%, and 0.1%. Next, the continuous growth of Sa240 was monitored by means of absorbance at 610 nm for 24 hours at 37°C.

### Real-Time Eradication of Biofilms

To evaluate the effect of different nanoparticles on *S. aureus* Sa240 biofilms preformed for 6 hours, xCELLigence Real-Time Analyzer was used according to the manufacturer's instructions. Preformed biofilm eradication experiments were performed as previously described (Reference 51), expressing biofilm growth as cell index (CI), which correlates with the total mass of biofilms. Briefly, *S. aureus* 240 was cultivated overnight in TSB medium. One hundred microliters of TSB-glu were used for background measurements (in triplicate for each condition).

### Confocal Laser Microscopy

To show how the nanoparticles interact with the exopolysaccharide of the biofilm, Alexa Fluor 488 fluorescent conjugate which binds to N-acetylneuramic acid and the polysaccharide adhesin involved in biofilm formation was used. Briefly, *S. aureus* 240 biofilms were cultured by means of adding 175 μl of bacterial suspension in the corresponding wells of IBIDI 80827 μ-Slide 8-well plates and biofilms were cultured at 37°C for 6 h as previously described, in the presence of 50 μl of Alexa Fluor 488 fluorescent conjugate (0.13 mg ml-1). Subsequently, the supernatant was removed and the biofilm was washed several times with TBS. After that, 1 mg ml-1 of **NM$_{VF}$, NM$_{VF}$** with 0.15% H$_2$O$_2$, and 0.15% H$_2$O$_2$ were added to the corresponding wells and the Sa240 biofilms were cultured for 24 h more.

To quantify living cells within bacterial biofilms after treatment with 1 mg ml$^{-1}$ **NM$_{VF}$, NM$_{VF}$** with 0.15% H$_2$O$_2$, and 0.15% H$_2$O$_2$, the SYTO9 fluorescent dye that binds to microbial DNA was used. Biofilms were grown as described above. Thereafter, treatments were added to the corresponding wells and the Sa240 biofilms were cultured for 24 hours more. Subsequently, the supernatant was discarded, the biofilms were carefully rinsed with PBS to remove loose cells and stained with 200 μl of SYTO9 solution (3 μl of SYTO9 working solution in 2 ml sterile water) for 20 min in the dark. After biofilm staining, excess labeling was removed by washing gently with water. Subsequently, the acquisition of confocal microscopy images was performed. To acquire the signals, 679 nm excitation laser and 702 nm emission filters were used for the Alexa Fluor 680-dextran conjugate, and 488 nm laser and 505-550 emission filter were used for SYTO9.

**Biofilm Micromorphology 24 h after Treatment**

Scanning electron microscopy (SEM) was used to evaluate the spatial structure of the biofilm after treatments. The overnight culture of *S. aureus* 240 was adjusted to an $OD_{600}$ = 0.0875 and the biofilms were cultured on xCELLigence E-plates for 24 h. After that, the biofilms were treated with $NM_{VF}$ in the presence and absence of $H_2O_2$, VF, and V for 6 hours. The supernatants were then discarded and the biofilms were gently washed with PBS buffer to remove non-adhered cells. Before the observations, biofilm samples were fixed with Karnovsky's fixative for 4 hours, rinsed with PBS three times, and dehydrated using graded series of ethanol (30%-50%-70%) twice. Then, the samples were dried using CO2 critical point drying. Subsequently, the samples were observed under SEM applying an accelerating voltage range of 0.5 kV at x2,500 magnification.

**Statistical Analysis**

To study the differences between the Cls, a regression analysis was performed by means of a linear model after 24 h of biofilm growth, using the lm library in R Statistical Package version 1.0.7.1 (references 52 and 53). Statistical differences in the number of viable cells were evaluated using Student's t-test. Data was presented as mean $\pm$ SD of triplicates of three independent experiments for each condition (n=9). For comparisons between means in confocal microscopy analysis, standard one-way ANOVA and Dunnett's multiple comparison test (n = 3) were used. Values of $p < 0.05$ were considered significant.

EXAMPLES OF EMBODIMENTS OF THE INVENTION

**Example 1. Self-propulsion Ability**

The study of the ability of the nanomotor (mesoporous silica nanoparticle with a platinum self-propulsion system) to self-propel in real time provided a diffusive movement of 7.22 $\mu$m/s at the selected concentration of fuel (0.2% $H_2O_2$) for antimicrobial studies. Likewise, it was verified how when increasing the concentration of the fuel the registered trajectories increased, which represents a great advantage for the penetrability of the devices in the protein matrix of bacterial biofilms (see Figure 1).

**Example 2. Release of the Load Encapsulated in the Nanodevice under Specific Conditions**

It was confirmed that the reduction of the pH of the medium (up to 4.5) causes an almost total release of the load encapsulated in the nanomotor in only 60 minutes, due to the rupture of the inclusion complex formed by ficin modified with b-cyclodextrins, and the benzimidazole groups that functionalize the MSN. However, the release is not representative at physiological pH. This demonstrates the proper functioning of the nanomotor to promote a controlled and localized release of the drug inside the biofilm, due to the acidic pH of the biofilm, with the drug remaining encapsulated when the medium is basic (see Figure 2).

**Example 3. Release of the Load Encapsulated in the Nanodevice under Specific Conditions**

The operation of the developed nanodevice was validated in an *in vitro S. aureus* biofilm model. In this study, the antimicrobial effect of the different components that are part of the nanosystem (separately and together) was evaluated by adding them to the preformed S. *aureus* Sa240 biofilm and monitoring its growth in real time by means of cell impedance measurements with the xCELLigence device. As can be observed in Figure 3, the addition of the complete nanodevice comprising the Janus Pt-MSN nanomotor and the fuel (curve marked with dotted line f) on the preformed biofilm, a removal greater than 75% is observed. This result demonstrates that the device acts as a molecular drill disintegrating the protein part of the biofilm matrix, as a result of its nanometric size and improved proteolytic activity of ficin due to the autonomous movement of the nanomotor. Furthermore, the synergistic effect between the movement and the specific release of the drug inside the biofilm produces a significant increase in therapeutic efficacy, obtaining a reduction greater than 90% in the number of viable cells (quantified by colony counting), compared to control.

**EXAMPLE 4. Effect of Nanoparticles on Endodontic Biofilms**

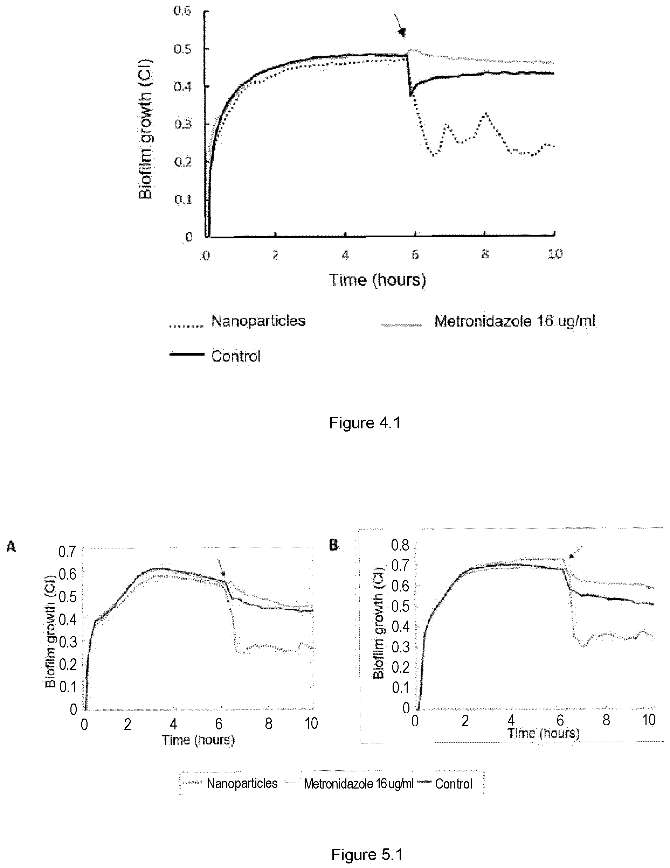

Figure 4.1. Disruption of endodontic biofilms using platinum and silica nanoparticles loaded with metronidazole. Endodontic biofilms were cultured in the xCELLigence system and after 6 hours of growth they were treated with 1 mg/ml of nanoparticles containing 2.25 $\mu$g of metronidazole, or with metronidazole in suspension at a concentration of 16 $\mu$g/ml, and compared to the untreated control (black curve). The black arrow indicates the time when the biofilm was treated. The biofilm comes from root canal samples extracted during a root canal and contains a complex microbial community formed by dozens of bacteria that cause this infection.

Endodontic biofilms from root canals were collected with sterile files and placed in 1 ml of VMG (III) transport medium (Dahlen *et al.,* 1993). Subsequently, the samples were transported to the laboratory where they were subjected to stirring for 3 minutes. Then, the files were extracted, the samples were centrifuged for 5 min at 10,000 rpm and the supernatant was discarded. The obtained pellet was resuspended with 1 ml of BHI (brain-heart infusion) culture medium and the samples were cultured in micro dilution plates of the xCELLigence impedance measurement system for 6 h, following the protocol described by Mira et al. 2019 (J Oral Microbiol). After 6 hours of growth, the endodontic biofilms were treated with 16 ug/ml of free metronidazole (the antibiotic concentration that corresponds to the peak serum concentration after oral administration of 500 mg metronidazole), or with 1 mg of platinum (Pt) nanoparticles loaded with metronidazole (final metronidazole concentration of 2.25 ug/ml). Figure 1 shows that metronidazole added in suspension (gray curve) induced up to 15% more biofilm formation compared to the untreated control (black curve). On the contrary, treatment with the nanoparticles resulted in up to 60% eradication of mature biofilm compared to the control, both after 7 and after 10 h of biofilm growth. These results indicate that the treatment of endodontic biofilms with nanoparticles loaded with a metronidazole concentration that is almost 8 times lower could generate better clinical results compared to the effect of the antibiotic administered in suspension in the traditional manner.

## EXAMPLE 5. Effect of Nanoparticles on Oral Biofilms

Figure 5.1. Effect of platinum and mesoporous silica nanoparticles loaded with metronidazole on preformed oral biofilms derived from saliva samples of two independent donors (A and B). The biofilms were grown in the xCELLigence system for 6 hours, and subsequently treated with metronidazole in suspension (concentration of 16 $\mu$g/ml) or with nanoparticles loaded with metronidazole (1 mg/ml of nanoparticles containing 2.25 $\mu$g metronidazole). The black arrows indicate the time when the treatment was added, whereas the black curves represent the untreated control. The biofilm comes from saliva samples and contains a complex microbial community made up of dozens of dental plaque-forming bacteria.

To investigate the effect of platinum (Pt) and mesoporous silica nanoparticles on polymicrobial oral biofilms (dental plaque), biofilms derived from the saliva of two independent donors were cultured in the xCELLigence system for 6 hours, following the protocol described by Mira et al. 2019 (J Oral Microbiol). Subsequently, free metronidazole (at a concentration of 16 ug/ml), or nanoparticles with metronidazole (1 mg/ml of nanoparticles containing 2.25 $\mu$g of metronidazole) was added to evaluate the effect on said biofilms. As shown in Figure 2, exposure of salivary biofilms to free metronidazole resulted in the induction of biofilm growth in both cases (A, B). On the contrary, when metronidazole was administered as nanoparticles with active platinum nanomotors, a reduction in biofilm biomass of between 70 and 73% was observed after 7 h of growth.

## EXAMPLE 6. Effect of Nanoparticles on *Candida albicans* Biofilms

Fungal biofilms represent a big problem in clinical practice, as they are associated with bloodstream infections, with infections from medical and implantable devices, and with certain clinical outbreaks. For example, *Candida albicans* forms thick, spacious biofilms in just 24 hours and these biofilms are made up of round yeast cells, pseudohyphae, and hyphae that contribute to the development of the exopolymeric matrix of the biofilm. Since mature fungal biofilms are extremely difficult to treat and there are only a limited number of conventional antifungals capable of inhibiting the formation of these biofilms, but none capable of eradicating same, there is a need to find new approaches to treat infections mediated by the biofilms of *Candida spp* and other fungal species such as *Aspergillus* or *Cryptococcus.*

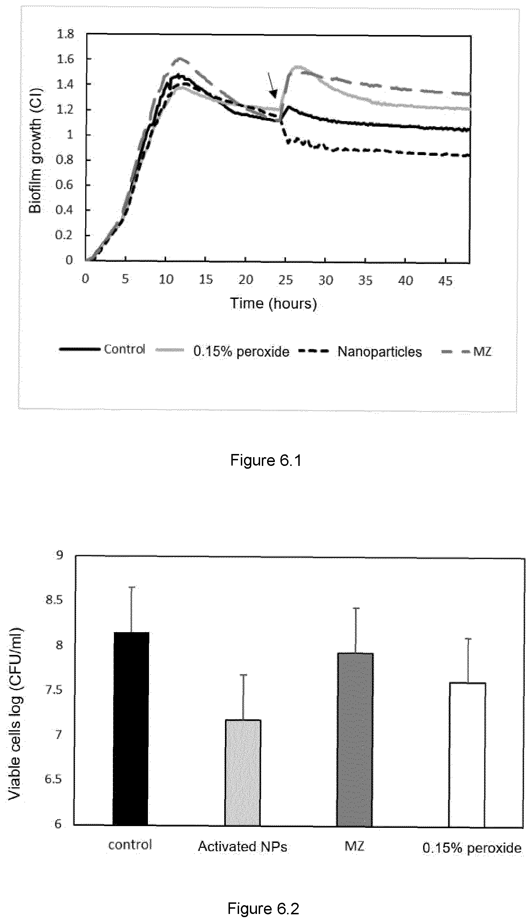

Figure 6.1. Effect of platinum and mesoporous silica nanoparticles on the eradication of mature *Candida albicans* biofilms. *C. albicans* biofilms were cultured in the micro dilution plates of the xCELLigence system for 24 h and then treated with a combination of free micafungin together with zymolyase (an enzyme with the ability to disperse biofilm-forming cells), indicated in the legend as MZ, or with nanoparticles loaded with the antifungal micafungin and anchored with the enzyme zymolyase. The movement of the nanoparticles was activated with 0.15% hydrogen peroxide, which was also included as an experiment control. The black curve represents the growth of the untreated biofilm and the black arrow indicates the time when the treatment was added.

As shown in , treatment of mature Figure 3.1*Candida albicans* biofilms at 24 h with the combination of the antifungal micafungin (1.5 $\mu$g/ml) plus the enzyme zymolyase (171 $\mu$g/ml), as well as the addition of 0.15% hydrogen peroxide, induced the formation of biofilms in both cases. On the contrary, nanoparticles activated with 0.15% hydrogen peroxide with the same concentrations of micafungin and zymolyase resulted in a reduction in biofilm biomass of up to 20% with respect to the untreated control. Furthermore, viable colony counts from each of the conditions of the biofilms grown at 24 hours (Figure 3.2) showed a 10-fold reduction in the number of viable cells when the biofilms were treated with activated nanoparticles that are loaded with micafungin and anchored with zymolyase. On the contrary, when micafungin plus zymolyase in suspension was added, this combination was not capable of reducing the number of viable cells, which suggests that nanoparticles could be used as a new approach to treat *Candida albicans* biofilm-mediated infections.

Figure 6.2. *Candida albicans* biofilm cell viability after t reatment with activated Pt and mesoporous silica nanoparticles, micafungin plus zymolyase in suspension (MZ), or 0.15% hydrogen peroxide, and the untreated control. Biofilms were treated after 24 h of biofilm growth and viable colony counts were performed 24 h later. The activity of the antifungal treatment was higher when applied in the form of activated nanoparticles.

## Bibliographic References

Dahlén G, Pipattanagovit P, Rosling B, Möller AJ. A comparison of two transport media for saliva and subgingival samples. Oral Microbiol Immunol. 1993 Dec;8(6):375-82. doi: 10.1111/j.1399-302x.1993.tb00614.x. PMID: 8152839.
Mira A, Buetas E, Rosier B, Mazurel D, Villanueva-Castellote Á, Llena C, Ferrer MD. Development of an in vitro system to study oral biofilms in real time through impedance technology: validation and potential applications. J Oral Microbiol. 2019 May 6;11(1):1609838. doi: 10.1080/20002297.2019.1609838. PMID: 31105900; PMCID: PMC6507917.

## Conclusions

In conclusion, the use of self-propelled Janus Pt-MSN nanomotors (nanoparticles comprising the Pt self-propulsion system, functionalized with ficin and CD and with a therapeutic agent encapsulated in the nanoparticle, such as the nanodevices of the invention) as a therapeutic strategy will facilitate the efficacy of antibiotics and antifungals for the treatment of infections associated with bacterial and fungal biofilms, respectively. Furthermore, as a result of the targeting effect of the nanomotors, a lower antibiotic dose would be needed to maintain therapeutic concentrations at the site of infection and it would be a more immediate treatment option that is more economical and presents fewer side effects and discomfort for patients, generating at the same time a tool for the fight against the major global health problem of bacterial multi resistance. Likewise, it would reduce prolonged hospital stays by improving the quality of life of the patient, with subsequent savings in direct and indirect healthcare costs.

## Claims

1. A nanodevice comprising a nanoparticle, a molecular drill attached to the surface of the nanoparticle, and a self-propulsion system attached to the surface of the nanoparticle.

2. The nanodevice according to claim 1, wherein the nanodevice further comprises at least one therapeutic agent in contact with the surface of the nanoparticle.

3. The nanodevice according to claim 2, wherein the therapeutic agent is attached to the surface of the nanoparticle, or comprised in the pores of the nanoparticle.

4. The nanodevice according to any of claims 1-3, wherein the nanoparticle is of an inorganic or organic type.

5. The nanodevice according to claim 4, wherein the inorganic nanoparticle is porous and is selected from the list consisting of MCM-41, MCM-48, MCM-50, and SBA, wherein the SBA nanoparticle is preferably SBA-15.

6. The nanodevice according to claim 5, wherein the porous nanoparticle is MCM-41.

7. The nanodevice according to claim 4, wherein the organic nanoparticle is a polymeric nanoparticle, a liposome, a micelle, a dendrimer, or a protocell.

8. The nanodevice according to any of claims 1-7, wherein the molecular drill comprises at least one compound with catalytic activity attached to a molecular complex that is attached to the surface of the nanoparticle.

9. The nanodevice according to claim 8, wherein the at least one compound with catalytic activity is selected from the list consisting of an enzyme, a mucolytic agent, a lipopeptide, chitosan, cis-2-decanoic acid (C2DA), nitric oxide, rhamnolipids, cerium IV, and combinations thereof.

10. The nanodevice according to claim 9, wherein the at least one compound with catalytic activity is an enzyme selected from the list consisting of a protease, DNAse, glycosidase, amylase, cellulase, dispersin B, pancreatin, and combinations thereof.

11. The nanodevice according to claim 10, wherein the protease is selected from the list consisting of ficin, proteinase K, trypsin, lysostaphin, peptidase M16, and combinations thereof.

12. The nanodevice according to claim 11, wherein the protease is ficin.

13. The nanodevice according to claim 8, wherein the at least one compound with catalytic activity is a mucolytic agent selected from the list consisting of ambroxol, N-acetylcysteine (NAC), and combinations thereof.

14. The nanodevice according to any of claims 8-13, wherein the molecular complex of the molecular drill comprises a cyclodextrin inclusion complex **charac-**

**terized in that** a hydrophobic molecule is housed inside the non-hydrophilic interior of the cyclodextrin toroid.

15. The nanodevice according to claim 14, wherein the cyclodextrin is α-cyclodextrin, β-cyclodextrin, or Y-cyclodextrin.

16. The nanodevice according to claim 15, wherein the hydrophobic molecule is an imidazole derivative or an aniline that deprotonates at pH equal to or less than 6.5, preferably equal to or less than 5.

17. The nanodevice according to claim 16, wherein the imidazole derivative is benzimidazole, bifonazole, ketoconazole, tioconazole, miconazole, itraconazole.

18. The nanodevice according to any of the preceding claims, wherein the self-propulsion system comprises a metal selected from the list consisting of Pt, Ag, Ir, Ni, Mg, and combinations thereof, the mineral $MnO_2$, and/or an enzyme selected from the list consisting of urease, glucose oxidase, and combinations thereof.

19. The nanodevice according to claim 18, wherein the metal is Pt.

20. The nanodevice according to any of claims 18-19, wherein the metal or mineral is in the form of a nanoparticle or surrounds part of the surface of the nanoparticle of the nanodevice.

21. The nanodevice according to any of claims 2-20, wherein the therapeutic agent is an antimicrobial compound and/or an antiseptic compound.

22. The nanodevice according to claim 21, wherein the antimicrobial compound is an antibiotic or an antifungal.

23. The nanodevice according to claim 22, wherein the antibiotic is selected from the list consisting of vancomycin, cloxacillin, levofloxacin, gentamicin, rifampicin, clarithromycin, cefotaxime, imipenem, moxifloxacin, linezolid, ciprofloxacin, tobramycin, ceftazidime, colistin, ciperacillin-tazobactam, imipenem, meropenem, amoxicillin, amoxicillin-clavulanic acid, metronidazole, clindamycin, azithromycin, dalbavancin, and combinations thereof.

24. The nanodevice according to claim 22, wherein the antifungal is selected from the list consisting of fluconazole, voriconazole, micafungin, caspofungin, posaconazole, anidulafungin, clotrimazole, and combinations thereof.

25. The nanodevice according to claim 22, wherein the antiseptic is selected from the list consisting of chlorhexidine, cetylpyridinium chloride, povidone iodine, sodium hypochlorite, and combinations thereof.

26. A pharmaceutical composition comprising the nanodevice according to any of claims 1-25.

27. The nanodevice or pharmaceutical composition according to any of claims 1-26 for use as a medicinal product.

28. The nanodevice or pharmaceutical composition according to any of claims 1-26 for use in the treatment of an infection in a subject, **characterized in that** the infected area in the subject comprises a biofilm produced by microorganisms that have invaded said infected area.

29. The nanodevice or pharmaceutical composition for use according to claim 28, wherein the microorganisms are at least one bacterium, and/or a fungus.

30. The nanodevice or pharmaceutical composition for use according to claim 29, wherein at least one bacterium is of the genus *Staphylococcus* spp., and/or *Pseudomonas* spp.

31. The nanodevice or pharmaceutical composition for use according to claim 30, wherein the bacterium *Staphylococcus* spp. is of the species *Staphylococcus aureus (S. aureus), Staphylococcus epidermidis (S. epidermidis), Staphylococcus lugdunensis (S. lugdunensis), Staphylococcus saprophyticus (S. saprophyticus, Staphylococcus hominis (S. hominis), Staphylococcus haemolyticus (S. haemolyticus), Staphylococcus capitis (S. capitis), Staphylococcus capitis (S. capitis capitis), and/or Staphylococcus warneri (S. warneri)*.

32. The nanodevice or pharmaceutical composition for use according to claim 30, wherein the bacterium is of the species *S. aureus.*

33. The nanodevice or pharmaceutical composition for use according to claim 30, wherein the bacterium *Pseudomonas* spp. is of the species *Pseudomonas aeruginosa (P. aeruginosa), Pseudomonas pseudomallei (P. pseudomallei), Pseudomona mirabilis (P. mirabilis), Pseudomonas oryzihabitans (P. oryzihabitans), Pseudomonas fluorecens (P. fluorecens), Pseudomonas putida (P. putida), Pseudomonas stutzeri (P. stutzeri), and/or Pseudomonas pickettii (P. pickettii).*

34. The nanodevice or pharmaceutical composition for use according to claim 29, wherein the at least one fungus is of the genus *Candida* spp., preferably of the species *Candida albicans (C. albicans), Candida Auris (C. Auris), Candida glabrata (C. galabatra), Candida parapsilosis (C. parapsilosis), and/or Candida tropicalis (C. tropicalis),* or of the genus *Aspergillus spp.,* and/or *Cryptococcus spp.*

35. The nanodevice or pharmaceutical composition for use according to any of claims 29-30, wherein the at least one bacterium is of the genus *Staphylococcus* spp. or the nanodevice or pharmaceutical composition for use according to any of claims 31-33, wherein the therapeutic agent comprised in the nanodevice is an antibiotic selected from the list consisting of vancomycin, cloxacillin, levofloxacin, gentamicin, rifampicin, clarithromycin, cefotaxime, imipenem, moxifloxacin, linezolid, dalbavancin, and combinations thereof.

36. The nanodevice or pharmaceutical composition for use according to claim 35, wherein the at least one bacterium is of the species *S. aureus* and the antibiotic is vancomycin.

37. The nanodevice or pharmaceutical composition for use according to claim 29-30, wherein the at least one bacterium is of the genus *Pseudomonas* spp. or the nanodevice or pharmaceutical composition for use according to claim 33, wherein the therapeutic agent comprised in the nanodevice is an antibiotic selected from the list consisting of ciprofloxacin, tobramycin, ceftazidime, colistin, ciperacillin-tazobactam, imipenem, meropenem, amoxicillin, amoxicillin-clavulanic acid, metronidazole, clindamycin, azithromycin, and combinations thereof.

38. The nanodevice or pharmaceutical composition for use according to claim 29 or 34, wherein the therapeutic agent comprised in the nanodevice is an antifungal selected from the list consisting of fluconazole, voriconazole, micafungin, caspofungin, posaconazole, anidulafungin, clotrimazole, and combinations thereof.

39. The nanodevice or pharmaceutical composition for use according to any of claims 29-38, wherein the infection is part of a pathology selected from the list consisting of endodontic infection, caries, periodontitis, halitosis, vaginosis, onychomycosis, mastitis, abscesses, and combinations thereof.

40. The nanodevice or pharmaceutical composition for use according to claim 39, wherein the condition is an endodontic infection.

41. The nanodevice or pharmaceutical composition for use according to any of claims 28-40, wherein the nanodevice or the pharmaceutical composition and a fuel are administered to the infected area of the subject.

42. The nanodevice or pharmaceutical composition for use according to claim 40, wherein the self-propulsion system of the nanodevice comprises a metal selected from the list consisting of Pt, Ag, Ir, Ni, Mg, and combinations thereof, and/or the mineral $MnO_2$, and wherein the fuel is hydrogen peroxide.

43. The nanodevice or pharmaceutical composition for use according to claim 41, wherein the self-propulsion system of the nanodevice comprises the enzyme urease and the fuel is urea.

44. The nanodevice or pharmaceutical composition for use according to claim 41, wherein the self-propulsion system of the nanodevice comprises the enzyme glucose oxidase and the fuel is glucose.

45. The nanodevice or pharmaceutical composition for use according to any of claims 41-44, wherein the nanodevice or pharmaceutical composition and the fuel are co-administered.

46. The nanodevice or pharmaceutical composition for use according to any of claims 41-45, wherein the amount of nanodevice administered is 0.25-5 mg/ml, preferably 0.5-1.5 mg/ml, with respect to the volume of a solution in which it is administered, wherein the solution is preferably distilled water.

47. The nanodevice or pharmaceutical composition for use according to any of claims 41-46, wherein the amount of fuel administered is 0.5-5 $\mu$l/ml, preferably 0.5-2.5 $\mu$l/ml with respect to the volume of a solution in which it is administered, wherein the solution is preferably distilled water.

48. The nanodevice or pharmaceutical composition for use according to any of claims 28-40, wherein the self-propulsion system of the nanodevice comprises the enzyme glucose oxidase and wherein the nanodevice or the pharmaceutical composition is administered to the infected area of the subject and wherein no fuel is administered, wherein said fuel is preferably glucose.

49. The nanodevice or pharmaceutical composition for use according to any of claims 28-40, wherein the self-propulsion system of the nanodevice comprises Zn and wherein the nanodevice or the pharmaceutical composition is administered to the infected area of the subject and wherein no fuel is administered.

50. Use of the nanodevice or of the pharmaceutical composition according to any of claims 1-26 to reduce the number of living microorganisms in a sample *in vitro* or on the surface of an inert material *ex*

*vivo,* wherein said microorganisms have formed a biofilm.

51. A kit comprising the nanoparticle, the molecular drill, the therapeutic agent, and the self-propulsion system comprised in the nanodevice according to any of claims 1-25.

52. The kit according to claim 51, further comprising a fuel.

53. The kit according to any of claims 51-52, further comprising a pharmaceutically acceptable adjuvant.

Fig. 1

Fig. 2

Fig. 3

Figure 4.1

Figure 5.1

Figure 6.1

Figure 6.2

## INTERNATIONAL SEARCH REPORT

| International application No |
|---|
| PCT/ES2023/070337 |

**A. CLASSIFICATION OF SUBJECT MATTER**

INV. A61K9/00    A61K9/16    A61K47/02    A61K47/40    A61K47/42

ADD.

According to International Patent Classification (IPC) or to both national classification and IPC

**B. FIELDS SEARCHED**

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

EPO-Internal

**C. DOCUMENTS CONSIDERED TO BE RELEVANT**

| Category* | Citation of document, with indication, where appropriate, of the relevant passages | Relevant to claim No. |
|---|---|---|
| X | Sanchez Samuel ET AL: "Sanchez et al. reported the use of self-propelled rolled-up microtubes to drill and embed themselves into biomaterials such as cells", 2 nd International Conference on Nanotechnologies and Biomedical Engineering, Chisinau, Republic of Moldova, April 18-20, 2013, 18 April 2013 (2013-04-18), pages 426-428, XP093080606, Retrieved from the Internet: URL:http://icnbme2015.sibm.md/files/icnbme _2013_proc/articles/SECTION%20Biomedical%2 0Instrumentation%20and%20Devices/150.pdf [retrieved on 2023-09-11] | 1-27, 51-53 |
| A | abstract | 28-50 |

-/--

[X] Further documents are listed in the continuation of Box C.     [ ] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 12 September 2023 | 26/09/2023 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Konter, Jörg |

Form PCT/ISA/210 (second sheet) (April 2005)

34

**EP 4 529 916 A1**

## INTERNATIONAL SEARCH REPORT

International application No

PCT/ES2023/070337

**C(Continuation).** DOCUMENTS CONSIDERED TO BE RELEVANT

| Category* | Citation of document, with indication, where appropriate, of the relevant passages | Relevant to claim No. |
|---|---|---|
| X | SOKOLOV ILYA L ET AL: "Smart materials on the way to theranostic nanorobots: Molecular machines and nanomotors, advanced biosensors, and intelligent vehicles for drug delivery", BIOCHIMICA ET BIOPHYSICA ACTA, ELSEVIER, AMSTERDAM, NL, vol. 1861, no. 6, 24 January 2017 (2017-01-24), pages 1530-1544, XP085110722, ISSN: 0304-4165, DOI: 10.1016/J.BBAGEN.2017.01.027 | 1-27, 51-53 |
| A | page 1536, right column, bottom paragraph to page 1537 ----- | 28-50 |
| X | SHUANGHU WANG ET AL: "The Application of Micro- and Nanomotors in Classified Drug Delivery", CHEMISTRY - AN ASIAN JOURNAL, WILEY-VCH, HOBOKEN, USA, vol. 14, no. 14, 2 May 2019 (2019-05-02), pages 2336-2347, XP072429062, ISSN: 1861-4728, DOI: 10.1002/ASIA.201900274 | 1-27, 51-53 |
| A | figures 1-8 ----- | 28-50 |
| X | SAMUEL SÁNCHEZ ET AL: "Chemically Powered Micro- and Nanomotors", ANGEWANDTE CHEMIE INTERNATIONAL EDITION, VERLAG CHEMIE, HOBOKEN, USA, vol. 54, no. 5, 12 December 2014 (2014-12-12), pages 1414-1444, XP072068745, ISSN: 1433-7851, DOI: 10.1002/ANIE.201406096 | 1-27, 51-53 |
| A | figures 1-25 ----- | 28-50 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PINTO R.M. et al.** *Frontiers in Microbiology*, 2020, vol. 11, 952
- **METCALF et al.** *J Wound Care*, 2014, vol. 23 (3), 137-42
- **TORREGROSA JERE L.** ; **VERDÚ SORIANO J.** Diagnostic methods for the identification of biofilm in chronic wounds. Systemic Review. Univ. Alicante, 2015
- **VERDÚ SORIANO J.** Diagnostic methods for the identification of biofilm in chronic wounds. Systemic Review. Univ. Alicante, 2015
- *Infectious Diseases and Clinical Microbiology*, 2011, vol. 29 (8)
- **BAKER et al.** *Sci. Adv.*, 2016, vol. 2, e1501632
- **MIRA et al.** *J Oral Microbiol*, 2019
- **DAHLÉN G** ; **PIPATTANAGOVIT P** ; **ROSLING B** ; **MÖLLER AJ**. A comparison of two transport media for saliva and subgingival samples.. *Oral Microbiol Immunol.*, December 1993, vol. 8 (6), 375-82
- **MIRA A** ; **BUETAS E** ; **ROSIER B** ; **MAZUREL D** ; **VILLANUEVA-CASTELLOTE Á** ; **LLENA C** ; **FERRER MD**. Development of an in vitro system to study oral biofilms in real time through impedance technology: validation and potential applications.. *J Oral Microbiol.*, 06 May 2019, vol. 11 (1), 1609838